# EUROPEAN PATENT APPLICATION

(11) **EP 4 606 820 A2**
(43) Date of publication of application: **27.08.2025**
(21) Application number: 25172734.3
(22) Date of filing: 30.11.2016
(51) Int. Cl.: C07K 16/00

(54) **OPTIMIZED RATIOS OF AMINO ACIDS AND SUGARS AS AMORPHOUS STABILIZING COMPOUNDS IN PHARMACEUTICAL COMPOSITIONS CONTAINING HIGH CONCENTRATIONS OF PROTEIN-BASED THERAPEUTIC AGENTS**

(30) Priority: 30.11.2015 US 201562260677 P
(62) Divisional of application: 16871377.4
(71) Applicant: Medimmune, LLC, Gaithersburg, Maryland 20878 (US)
(72) Inventor: PATEL, Sajal M., Gaithersburg (US); PANSARE, Swapnil K., Gaithersburg (US)
(74) Representative: Mathys & Squire

(57) **Abstract**

The present invention relates to improved pharmaceutical compositions that contain high concentrations of one or more protein biomolecule(s). In particular, the invention relates to pharmaceutical compositions that include an optimized ratio of protein biomolecule to an amorphous stabilizing compound or compounds, especially a sugar, such as sucrose, trehalose, glucose, lactose or sorbitol, or mixtures thereof, or one or more amino acid molecules such as arginine, alanine, glycine, lysine or proline, or derivatives and salts thereof, or mixtures thereof. The inclusion of such amorphous stabilizing compound(s), at such optimized ratio, provides acceptable long-term stability of the protein biomolecule, and facilitates shorter lyophilization time, more specifically shorter drying time, even more specifically shorter primary drying time.

## Description

### Field Of The Invention

The present invention relates to improved pharmaceutical compositions that contain high concentrations of one or more protein biomolecule(s). In particular, the invention relates to pharmaceutical compositions that include an optimized ratio of protein biomolecule to an amorphous stabilizing compound or compounds, especially a sugar, such as sucrose, trehalose, glucose, lactose or sorbitol, or mixtures thereof, or one or more amino acid molecules such as arginine, alanine, glycine, lysine or proline, or derivatives and salts thereof, or mixtures thereof. The inclusion of such amorphous stabilizing compound(s), at such optimized ratio, provides acceptable long-term stability of the protein biomolecule, and facilitates shorter lyophilization time, more specifically shorter drying time, even more specifically shorter primary drying time.

### Background Of The Invention

Protein-based therapeutic agents (e.g., hormones, enzymes, cytokines, vaccines, immunotherapeutics, *etc.)* are becoming increasingly important to the management and treatment of human disease. As of 2014, more than 60 such therapeutics had been approved for marketing, with approximately 140 additional drugs in clinical trial and more than 500 therapeutic peptides in various stages of preclinical development (Fosgerau, K. et al. (2014) "Peptide Therapeutics: Current Status And Future Directions," Drug Discov. Today 20(1):122-128; Kaspar, A.A. et al. (2013) "Future Directions For Peptide Therapeutics Development," Drug Discov. Today 18:807-817).

One impediment to the use of such therapeutics is the physical instability that is often encountered upon their storage (US Patent No. 8,617,576; PCT Publications No. WO 2014/100143 and 2015/061584; Balcão, V.M. et al. (2014) "Structural And Functional Stabilization Of Protein Entities: State-Of-The-Art," Adv. Drug Deliv. Rev. (Epub.): doi: 10.1016/j.addr.2014.10.005; pp. 1-17; Maddux, N.R. et al. (2011) "Multidimensional Methods For The Formulation Of Biopharmaceuticals And Vaccines," J. Pharm. Sci. 100:4171-4197; Wang, W. (1999) "Instability, Stabilization, And Formulation Of Liquid Protein Pharmaceuticals," Int. J. Pharm. 185:129-188; Kristensen, D. et al. (2011) "Vaccine Stabilization: Research, Commercialization, And Potential Impact," Vaccine 29:7122-7124; Kumru, O.S. et al. (2014) "Vaccine Instability In The Cold Chain: Mechanisms, Analysis And Formulation Strategies," Biologicals 42:237-259). Such instability may comprise multiple aspects. A protein-based therapeutic agent may, for example experience **operational instability,** such as an impaired ability to survive processing operations *(e.g.,* sterilization, lyophilization, cryopreservation, *etc.).* Additionally or alternatively, proteins may experience **thermodynamic instability** such that a desired secondary or tertiary conformation is lost or altered upon storage. A further, and especially complex problem, lies in the stabilization of therapeutic agents that comprise multimeric protein subunits, with dissociation of the subunits resulting in the inactivation of the product. Kinetic instability is a measure of the capacity of a protein to resist irreversible changes of structure in *in vitro* non-native conditions. Protein aggregation and the formation of inclusion bodies is considered to be the most common manifestation of instability, and is potentially encountered in multiple phases of product development (Wang, W. (2005) "Protein Aggregation And Its Inhibition In Biopharmaceutics," Int. J. Pharm. 289:1-30; Wang, W. (1999) "Instability, Stabilization, And Formulation Of Liquid Protein Pharmaceuticals," Int. J. Pharm. 185:129-188; Arakawa, T. et al. (1993) "Factors Affecting Short-Term And Long-Term Stabilities Of Proteins," Adv. Drug Deliv. Rev. 10:1-28; Arakawa, T. et al. (2001) "Factors Affecting Short-Term And Long-Term Stabilities Of Proteins," Adv. Drug Deliv. Rev. 46:307-326). Such issues of instability can affect not only the efficacy of the therapeutic but its immunogenicity to the recipient patient. Protein instability is thus one of the major drawbacks that hinders the use of protein-based therapeutic agent (Balcão, V.M. et al. (2014) "Structural And Functional Stabilization Of Protein Entities: State-Of- The-Art," Adv. Drug Deliv. Rev. (Epub.): doi: 10.1016/j.addr.2014.10.005; pp. 1-17).

Stabilization of protein-based therapeutic agents entails preserving the structure and functionality of such agents, and has been accomplished by establishing a thermodynamic equilibrium between such agents and their (micro)environment (Balcão, V.M. et al. (2014) "Structural And Functional Stabilization Of Protein Entities: State-Of- The-Art," Adv. Drug Deliv. Rev. (Epub.): doi: 10.1016/j.addr.2014.10.005; pp. 1-17). One approach to stabilizing protein-based therapeutic agents involves altering the protein to contain additional covalent (e.g., disulfide) bonds so as to increase the enthalpy associated with a desired conformation. Alternatively, the protein may be modified to contain additional polar groups so as to increase its hydrogen bonding with solvating water molecules (Mozhaev, V.V. et al. (1990) "Structure-Stability Relationships In Proteins: A Guide To Approaches To Stabilizing Enzymes," Adv. Drug Deliv. Rev. 4:387-419; Iyer, P.V. et al. (2008) "Enzyme Stability And Stabilization - Aqueous And Non-Aqueous Environment," Process Biochem. 43:1019-1032).

A second approach to stabilizing protein-based therapeutic agents involves reducing the chemical activity of the water present in the protein's microenvironment, for example by freezing the water, adding specific solutes, or lyophilizing the pharmaceutical composition (see, *e.g.,* Castronuovo, G. (1991) "Proteins In Aqueous Solutions. Calorimetric Studies And Thermodynamic Characterization," Thermochim. Acta 193:363-390).

Employed solutes range from small molecular weight ions (e.g., salts, buffering agents) to intermediate sized solutes (e.g., amino acids, sugars) to larger molecular weight compounds (e.g., polymers, proteins) (Kamerzell, T.J. et al. (2011) "Protein-Excipient Interactions: Mechanisms And Biophysical Characterization Applied To Protein Formulation Development," Adv. Drug Deliv. Rev. 63:1118-1159).

For example, such solutes have included budesonide, dextran DMSO glycerol, glucose, inulin, lactose, maltose, mannitol, PEG, piroxicam, PLGA, PVA sorbitol, sucrose, trehalose and urea (Ohtake, S. et al. (2011) "Trehalose: Current Use and Future Applications," J. Pharm. Sci. 100(6):2020-2053; Willart, J.F. et al. (2008) "Solid State Amorphization of Pharmaceuticals," Molec. Pharmaceut. 5(6):905-920; Kumru, O.S. et al. (2014) "Vaccine Instability In The Cold Chain: Mechanisms, Analysis And Formulation Strategies," Biologicals 42:237-259; Somero, G.N. (1995) "Proteins And Temperature," Annu. Rev. Physiol. 57: 43-68; Sasahara, K. et al. (2003) "Effect Of Dextran On Protein Stability And Conformation Attributed To Macromolecular Crowding," J. Mol. Biol. 326:1227-1237; Jain, N.K. et al. (2014) "Formulation And Stabilization Of Recombinant Protein Based Virus-Like Particle Vaccines," Adv. Drug Deliv. Rev. (Epub.) doi: 10.1016/j.addr.2014.10.023; pp. 1-14; Kissmann, J. et al. (2011) "H1N1 Influenza Virus-Like Particles: Physical Degradation Pathways And Identification Of Stabilizers," J. Pharm. Sci. 100:634-645; Kamerzell, T.J. et al. (2011) "Protein-Excipient Interactions: Mechanisms And Biophysical Characterization Applied To Protein Formulation Development," Adv. Drug Deliv. Rev. 63:1118-1159).

Sugars such as sucrose and trehalose dihydrate are typically used as lyoprotectants and cryoprotectants in lyophilized therapeutic protein formulations to improve drug product stability, *e.g.,* for storage at 2-8 °C (US Patents No. 8,617,576 and 8,754,195). Trehalose, in particular, has been widely used as a stabilizing agent; it is used in a variety of research applications and is contained in several commercially available therapeutic products, including HERCEPTIN^{®}, AVASTIN^{®}, LUCENTIS^{®}, and ADVATE^{®} (Ohtake, S. et al. (2011) "Trehalose: Current Use and Future Applications," J. Pharm. Sci. 100(6):2020-2053).

The amino acids histidine, arginine, glutamate, glycine, proline, lysine and methionine have been mentioned as natural compounds that stabilize proteins. Human serum albumin (HSA) and gelatin have been mentioned as being protein stabilizers (US Patent No. 8,617,576; US Patent Publication No. 2015/0118249; Kamerzell, T.J. et al. (2011) "Protein-Excipient Interactions: Mechanisms And Biophysical Characterization Applied To Protein Formulation Development," Adv. Drug Deliv. Rev. 63:1118-1159; Kumru, O.S. et al. (2014) "Vaccine Instability In The Cold Chain: Mechanisms, Analysis And Formulation Strategies," Biologicals 42:237-259; Arakawa, T. et al. (2007) "Suppression Of Protein Interactions By Arginine: A Proposed Mechanism Of The Arginine Effects," Biophys. Chem. 127:1-8; Arakawa, T. et al. (2007) "Biotechnology Applications Of Amino Acids In Protein Purification And Formulations," Amino Acids 33:587-605; Chen, B. (2003) "Influence Of Histidine On The Stability And Physical Properties Of A Fully Human Antibody In Aqueous And Solid Forms," Pharm. Res. 20:1952-1960; Tian, F. et al. (2007) "Spectroscopic Evaluation Of The Stabilization Of Humanized Monoclonal Antibodies In Amino Acid Formulations," Int. J. Pharm. 335:20-31; Wade, A.M. et al. (1998) "Antioxidant Characteristics Of L-Histidine," J. Nutr. Biochem. 9:308-315; Yates, Z. et al. (2010) "Histidine Residue Mediates Radical-Induced Hinge Cleavage Of Human Iggl," J. Biol. Chem. 285:18662-18671; Lange, C. et al. (2009) "Suppression Of Protein Aggregation By L-Arginine," Curr. Pharm. Biotechnol. 10:408-414; Nakakido, M. et al. (2009) "To Be Excluded Or To Bind, That Is The Question: Arginine Effects On Proteins," Curr. Pharm. Biotechnol. 10:415-420; Shukla, D. et al. (2010) "Interaction Of Arginine With Proteins And The Mechanism By Which It Inhibits Aggregation," J. Phys. Chem. B 114:13426-13438; Pyne, A. et al. (2001) "Phase Transitions Of Glycine In Frozen Aqueous Solutions And During Freeze-Drying," Pharm. Res. 18:1448-1454; Lam, X.M. et al. (1997) "Antioxidants For Prevention Of Methionine Oxidation In Recombinant Monoclonal Antibody HER2," J. Pharm. Sci. 86:1250-1255; Maeder, W. et al. (2011) "Local Tolerance And Stability Up To 24 Months Of A New 20% Proline-Stabilized Polyclonal Immunoglobulin For Subcutaneous Administration," Biologicals 39:43-49; Kadoya, S. et al. (2010) "Freeze-Drying Of Proteins With Glass-Forming Oligosaccharide-Derived Sugar Alcohols," Int. J. Pharm. 389:107-113; Golovanov, A.P. et al. (2004) "A Simple Method For Improving Protein Solubility And Long-Term Stability, J. Am. Chem. Soc. 126:8933-8939).

Typically, a protein-to-stabilizer compound ratio of 1:1 or 1:2 (w/w) has been used to achieve optimal stability for lower protein concentrations (< 50mg/mL). However, for higher protein concentrations (≥ 50mg/mL), protein-to-stabilizer compound ratios in the 1:1 or 1:2 (w/w) range are less desirable. For example, such high sugar concentrations can result in high viscosity, which impose challenges during fill-finish operations and in drug-delivery and can require increased reconstitution times for lyophilized formulations. Moreover, the reconstituted formulations can exhibit high osmolality, far outside the desired isotonic range, especially if partial reconstitution is desired in order to achieve a higher protein concentration. Finally, high concentration protein formulations with protein-to-stabilizer compound ratios in the 1:1 or 1:2 (w/w) range can exhibit thermal characteristics that require unacceptably long lyophilization process times at much lower temperatures.

Thus, despite all of such advances, a need remains for formulations suitable for stabilizing protein-based pharmaceutical compositions such that the pharmaceutical compositions would exhibit improved viscosity and reconstitution times and enhanced stability, both in lyophilized / cryopreserved form and following reconstitution. The present invention is directed to this and other goals.

### Summary Of The Invention

The present invention relates to improved pharmaceutical compositions that contain high concentrations of one or more protein biomolecule(s). In particular, the invention relates to pharmaceutical compositions that include an optimized ratio of protein biomolecule to an amorphous stabilizing compound or compounds, especially a sugar, such as sucrose, trehalose, glucose, lactose or sorbitol, or mixtures thereof, or one or more amino acid molecules such as arginine, alanine, glycine, lysine or proline, or derivatives and salts thereof, or mixtures thereof. The inclusion of such amorphous stabilizing compound(s), at such optimized ratio, provides acceptable long-term stability of the protein biomolecule, and facilitates shorter lyophilization time, more specifically shorter drying time, even more specifically shorter primary drying time.

In detail, the invention concerns a pharmaceutical composition comprising a protein biomolecule as an active agent or component thereof, wherein:
(A) the composition is an aqueous solution that comprises:
   (1) a protein biomolecule;
   (2) a buffer; and
   (3) an amorphous stabilizing compound;
      wherein in the aqueous solution:
      (a) the protein biomolecule is present at a concentration of about 50 mg/mL or less, and the amorphous stabilizing compound is present at a total concentration of from about 0.1% (w/v) to about 2.5% (w/v); or
      (b) the protein biomolecule is present at a concentration of greater than about 50 mg/mL, and the amorphous stabilizing compound is present at a total concentration of from about 1% (w/v) to about 8.5% (w/v);
   or
(B) the composition is a lyophilisate of the aqueous solution that comprises the protein biomolecule at a concentration of about 50 mg/mL or less.

The invention additionally concerns the embodiment of such pharmaceutical composition wherein the protein biomolecule is present in the aqueous solution at a concentration of about 50 mg/mL or less, and the amorphous stabilizing compound is present at a total concentration of from about 0.1% (w/v) to about 2.5% (w/v).

The invention additionally concerns the embodiment of such pharmaceutical compositions wherein the amorphous stabilizing compound is present at a total concentration of about 0.5% (w/v), or is present at a total concentration of about 1% (w/v).

The invention additionally concerns the embodiment of such pharmaceutical compositions wherein the pharmaceutical composition is the lyophilisate.

The invention additionally concerns the embodiment of such pharmaceutical compositions wherein the pharmaceutical composition is the aqueous solution, and wherein the protein biomolecule is present in the composition at a concentration of greater than about 50 mg/mL to about 500 mg/mL, and the amorphous stabilizing compound is present at a total concentration of from about 1% (w/v) to about 8.5% (w/v).

The invention additionally concerns the embodiment of such pharmaceutical compositions wherein the protein biomolecule is present in the composition at a concentration of about 100 mg/mL, and the amorphous stabilizing compound is present at a total concentration of from about 1% (w/v) to about 8.5% (w/v), and especially wherein the amorphous stabilizing compound is present at a total concentration of about 1% (w/v).

The invention additionally concerns the embodiment of such pharmaceutical compositions wherein the protein biomolecule is present in the composition at a concentration of about 200 mg/mL, and the amorphous stabilizing compound is present at a total concentration of from about 1% (w/v) to about 8.5% (w/v), and especially wherein the amorphous stabilizing compound is present at a total concentration of about 2% (w/v).

The invention additionally concerns the embodiment of such pharmaceutical compositions wherein the amorphous stabilizing compound is a sugar, and particularly wherein the sugar is sucrose, trehalose, glucose, lactose or sorbitol, or a mixture thereof.

The invention additionally concerns the embodiment of such pharmaceutical compositions wherein the amorphous stabilizing compound is an amino acid, and particularly wherein the amino acid is arginine, alanine, lysine, proline or glycine, or a derivative or salt thereof, or any mixture thereof.

The invention additionally concerns the embodiment of such pharmaceutical compositions wherein the composition comprises at least two amorphous stabilizing compounds, and particularly wherein one of the at least two amorphous stabilizing compounds is a sugar and the other is an amino acid.

The invention additionally concerns the embodiment of such pharmaceutical compositions wherein the composition comprises at least two protein biomolecules.

The invention additionally concerns the embodiment of such pharmaceutical compositions wherein the protein biomolecule (or at least one of the at least two protein biomolecules) is an antibody or an antibody-based immunotherapeutic, enzyme, or a hormone/factor.

The invention additionally concerns the embodiment of such pharmaceutical compositions wherein the protein biomolecule (or at least one of the at least two protein biomolecules) is an antibody or an antibody-based immunotherapeutic, and the antibody is selected from the antibodies of **Table 1.**

The invention additionally concerns the embodiment of such pharmaceutical compositions wherein the protein biomolecule (or at least one of the at least two protein biomolecules) is a hormone/factor, and the hormone/factor is selected from the hormone/factors of **Table 2.**

The invention additionally concerns the embodiment of such pharmaceutical compositions wherein the pH of the pharmaceutical composition is from about 3 to about 11, from about 4 to about 9, from about 5 to about 8, from about 5 to about 7.5, preferably 6.0 or 7.4.

The invention additionally concerns the embodiment of such pharmaceutical compositions wherein the buffer is present in a range of from about 1 mM to 100 mM, about 10 mM to about 50 mM, about 20 mM to about 30 mM, or about 23 mM to about 27 mM.

The invention additionally concerns the embodiment of such pharmaceutical compositions wherein the buffer comprises histidine, phosphate, acetate, citrate, succinate, Tris, or a combination thereof, and particularly wherein the buffer is histidine / histidine-HCl.

The invention additionally concerns the embodiment of such pharmaceutical compositions wherein the pharmaceutical composition additionally comprises a non-ionic detergent, especially the non-ionic detergent is polysorbate-80 (PS-80). The invention additionally concerns the embodiment of such pharmaceutical composition wherein such is polysorbate-80 (PS-80) is present at a concentration of 0.02% (w/v).

The invention additionally concerns the embodiment of such pharmaceutical compositions wherein the primary drying time is reduced by 25%, by 30%, by 35%, by 40%, by 45%, by 50%, by 55%, by 60%, by 65%, by 70%, by 75%, by 80%, by 85%, by 90%, or by 95%.

The invention additionally concerns an ampoule, vial, syringe, cartridge or sachette that contains the any of the above-described pharmaceutical compositions.

The invention additionally concerns a method of treating a disease or disorder by administering any of the above-described pharmaceutical compositions to a subject.

The invention additionally concerns any of the above-described pharmaceutical compositions for use in medicine.

### Brief Description Of The Drawings

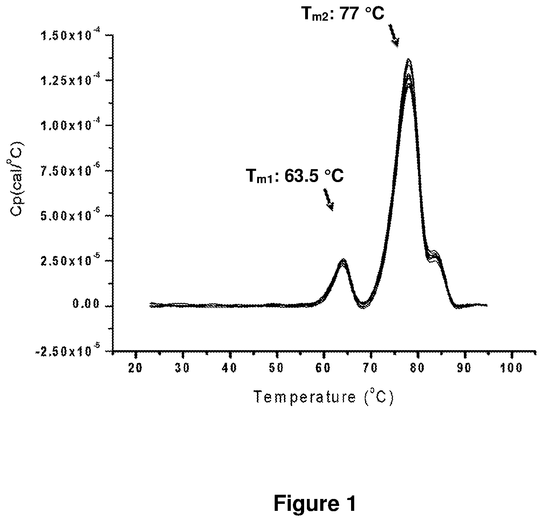

**Figure 1** shows the melting temperatures (Tₘ₁ and Tₘ₂) of pharmaceutical compositions containing 100 mg/mL, post-reconstitution, of an exemplary human IgG1 monoclonal antibody after lyophilized storage for 3 months at 40 °C at different protein-to-sugar ratios, as determined by differential scanning calorimetry (DSC). Lyophilized samples were reconstituted after 3 months storage at 40 °C and then diluted to 1 mg/mL protein concentration prior to DSC analysis.

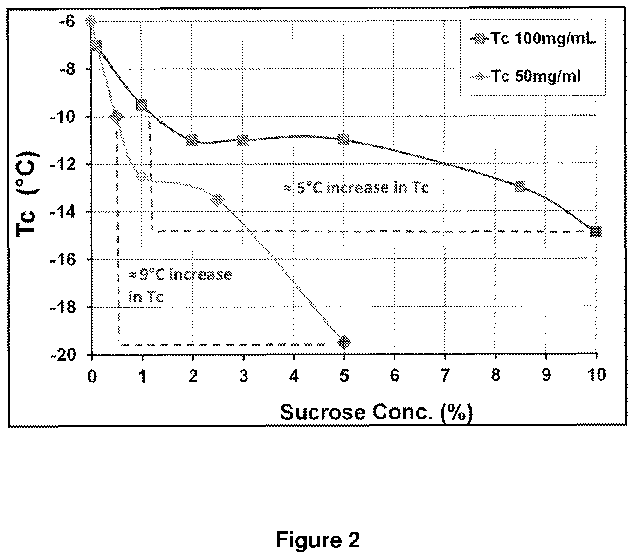

**Figure 2** shows the Onset of Collapse **(T_{c})** determined using Freeze Dry Microscopy (FDM) for a pharmaceutical composition containing 50 mg/mL or 100 mg/mL of an exemplary human IgG1 monoclonal antibody, as a function of the percentage of sucrose concentration.

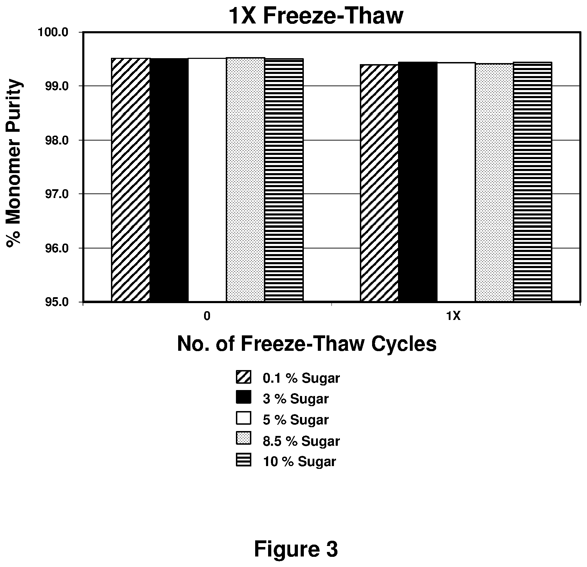

**Figure** 3 shows the percent monomer purity (as determined by high performance size-exclusion chromatography (HPSEC)) of a pharmaceutical composition containing 100 mg/mL of an exemplary human IgG1 monoclonal antibody at different sugar concentrations (w/v), in PETG containers before and after an uncontrolled freeze-thaw study.

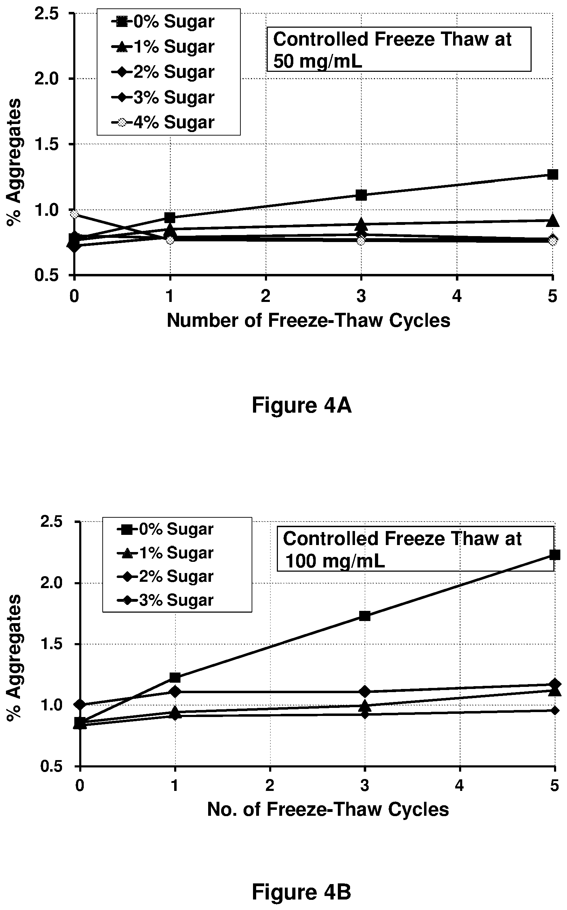

**Figures 4A-4B** show the % of aggregates in solution (as determined by high performance size-exclusion chromatography (HPSEC)) of a pharmaceutical composition containing 50 mg/mL
**(****Figure 4A****)** or 100 mg/mL **(****Figure 4B****)** of an exemplary human IgG1 monoclonal antibody at different sugar concentrations (w/v) before and after a controlled freeze-thaw performed in glass vial: freezing at -40 °C and thawing at 25 °C.

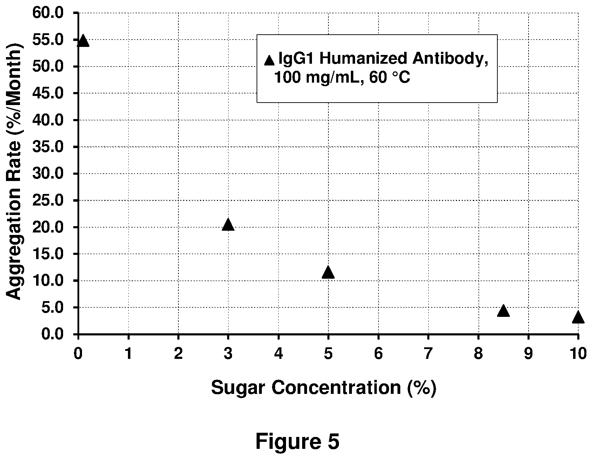

**Figure 5** shows the aggregation rate for lyophilized product (as determined by high performance size-exclusion chromatography (HPSEC)) of a pharmaceutical composition containing an exemplary human IgG1 monoclonal antibody at 100 mg/mL that had been stored at 60 °C for 8 days as a function of the percentage of sugar concentration (w/v).

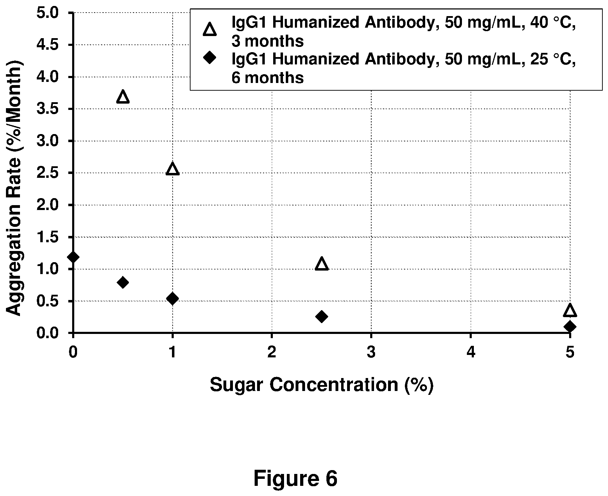

**Figure 6** shows the aggregation rate for lyophilized product (as determined by high performance size-exclusion chromatography (HPSEC)) of a pharmaceutical composition containing an exemplary human IgG1 monoclonal antibody (50 mg/mL) that had been stored at 40 °C or at 25 °C for 3 or 6 months, respectively, as a function of the percentage of sugar concentration (w/v).

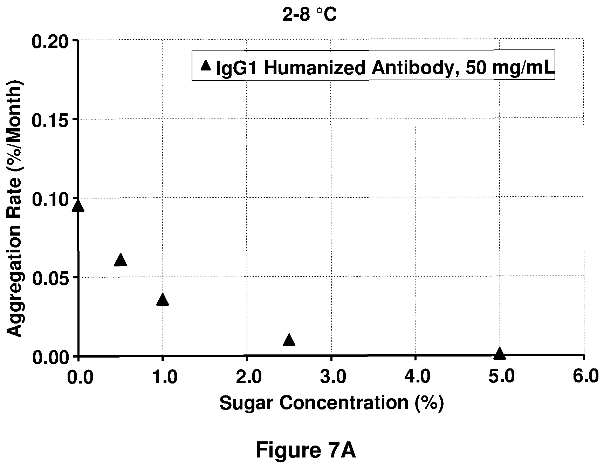

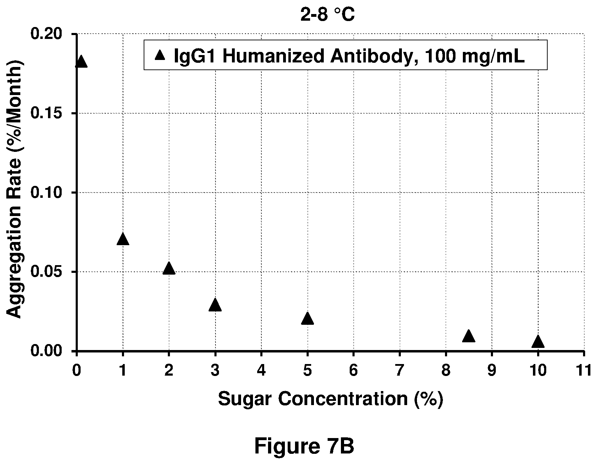

**Figures 7A-7B** show the aggregation rate for lyophilized product (as determined by high performance size-exclusion chromatography (HPSEC)) of a pharmaceutical composition containing 50 mg/mL **(****Figure 7A****)** or 100 mg/mL **(****Figure 7B****)** of an exemplary human IgG1 monoclonal antibody that had been stored at 2-8 °C for 12 months as a function of the percentage of sugar concentration (w/v).

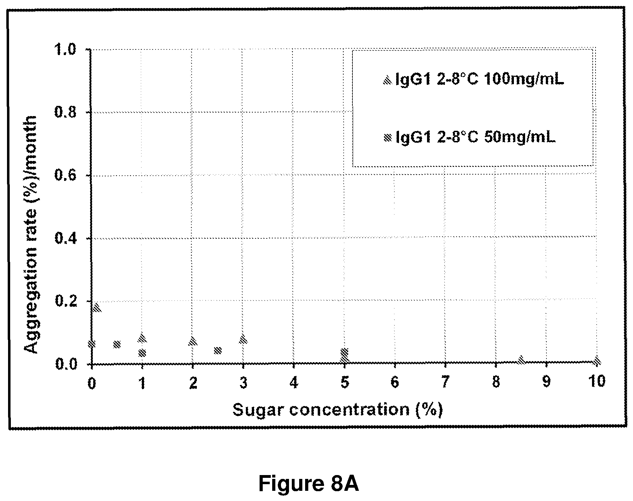

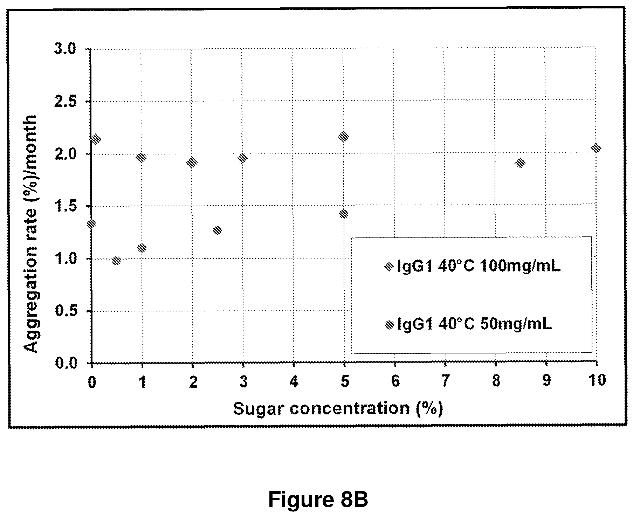

**Figures 8A-8B** shows the aggregation rate in solution (as determined by high performance size-exclusion chromatography (HPSEC)) of a pharmaceutical composition containing 50 or 100 mg/mL of an exemplary human IgG1 monoclonal antibody that had been stored at 2-8 °C for 9 months **(****Figure 8A****)** or containing 50 or 100 mg/mL of an exemplary human IgG1 monoclonal antibody that had been stored at 40 °C for 3 months **(****Figure 8B****)** as a function of the percentage of sugar concentration (w/v).

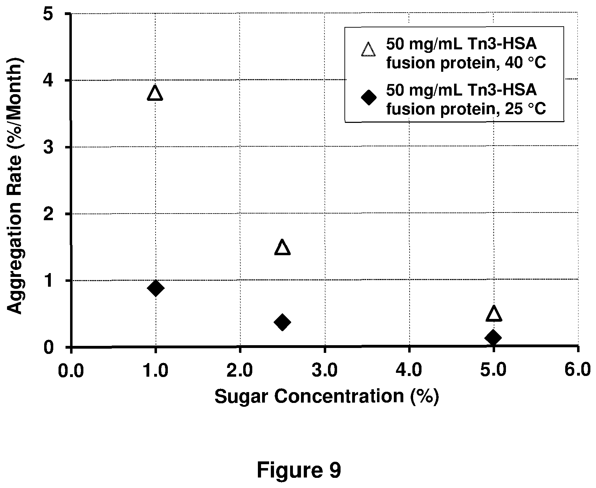

**Figure 9** shows the aggregation rate for lyophilized product (as determined by high performance size-exclusion chromatography (HPSEC)) of a pharmaceutical composition containing 50 mg/mL of an exemplary Tn3-HSA fusion protein that had been stored at 25 °C for 6 months or 40 °C for 3 months as a function of the percentage of sugar concentration (w/v).

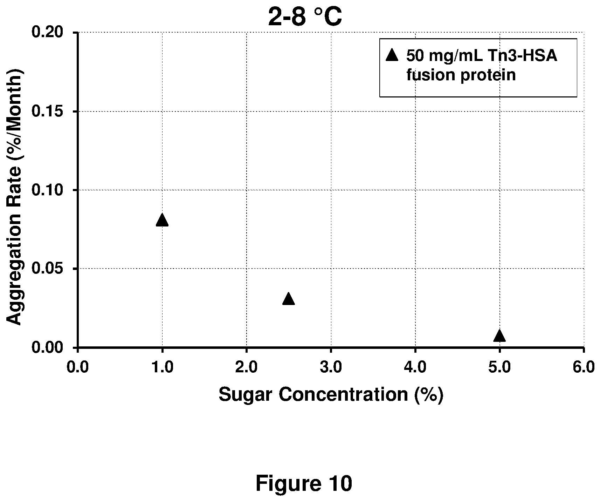

**Figure 10** shows the aggregation rate for lyophilized product (as determined by high performance size-exclusion chromatography (HPSEC)) of a pharmaceutical composition containing 50 mg/mL of an exemplary Tn3-HSA fusion protein that had been stored at 2-8 °C for 12 months as a function of the percentage of sugar concentration (w/v).

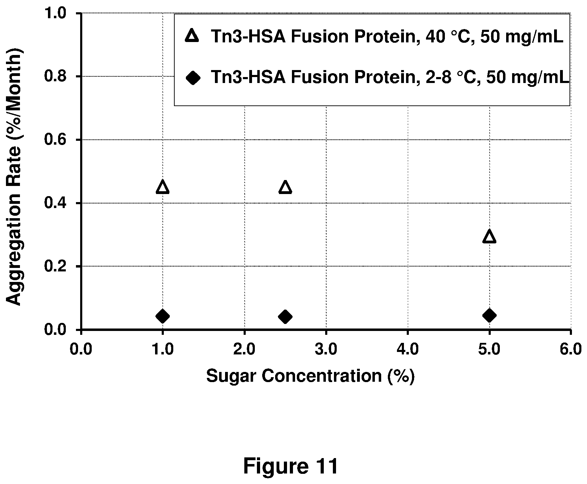

**Figure 11** shows the aggregation rate in solution (as determined by high performance size-exclusion chromatography (HPSEC)) of a pharmaceutical composition containing 50 mg/mL of an exemplary Tn3-HSA fusion protein that had been stored at 2-8 °C for 12 months or at 40 °C for 3 months as a function of the percentage of sugar concentration (w/v).

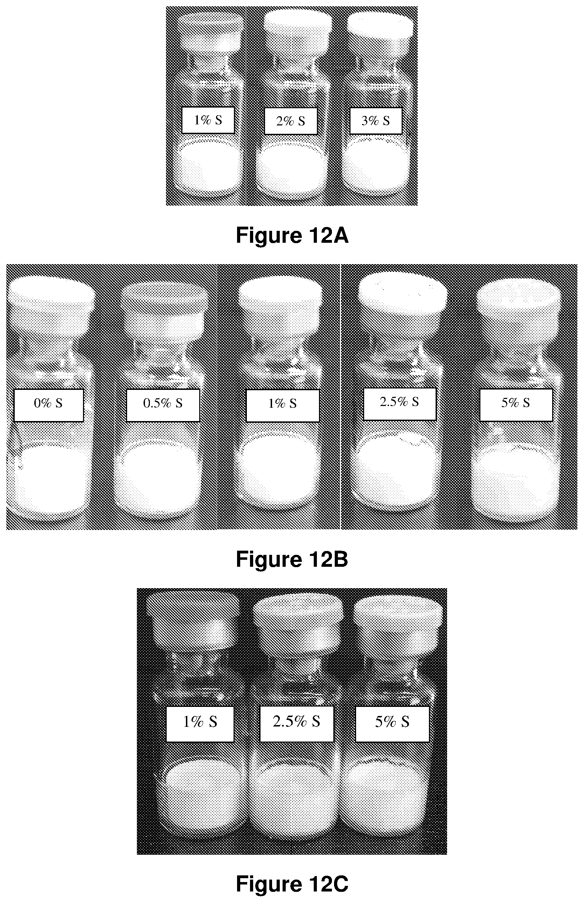

**Figures 12A-12C** show the visual appearance of the lyophilized cake for humanized IgG1 (at 50 and 100 mg/mL) and Tn3-HSA fusion protein (at 50 mg/mL) in formulations containing different levels of sucrose. **Figure 12A****:** Lyophilized cake for Humanized IgG1 at 100 mg/mL; **Figure 12B****:** Lyophilized cake for Humanized IgG1 at 50 mg/mL; **Figure 12C****:** Lyophilized cake for Humanized Tn3-HSA fusion protein at 50 mg/mL. "S" denotes sucrose.

### Detailed Description Of The Invention

The present invention relates to improved pharmaceutical compositions that contain high concentrations of one or more protein biomolecule(s). In particular, the invention relates to pharmaceutical compositions that include an optimized ratio of protein biomolecule to an amorphous stabilizing compound or compounds, especially a sugar, such as sucrose, trehalose, glucose, lactose or sorbitol, or mixtures thereof, or one or more amino acid molecules such as arginine, alanine, glycine, lysine or proline, or derivatives and salts thereof, or mixtures thereof. The inclusion of such amorphous stabilizing compound(s), at such optimized ratio, provides acceptable long-term stability of the protein biomolecule, and facilitates shorter lyophilization time, more specifically shorter drying time, even more specifically shorter primary drying time.

### I. The Pharmaceutical Compositions of the Present Invention

As used herein, the term **"pharmaceutical composition"** is intended to refer to a **"therapeutic"** medicament *(i.e.,* a medicament formulated to treat an existing disease or condition of a recipient subject) or a **"prophylactic"** medicament *(i.e.,* a medicament formulated to prevent or ameliorate the symptoms of a potential or threatened disease or condition of a recipient subject) containing one or more protein biomolecules as its active therapeutic or prophylactic agent or component. The pharmaceutical compositions of the present invention comprise one or more protein biomolecule(s) that serve(s) as an active agent or component of the composition. For therapeutic use, the pharmaceutical composition will contain and provide a **"therapeutically effective"** amount of the protein biomolecule(s), which is an amount that reduces or ameliorates the progression, severity, and/or duration of a disease or condition, and/or ameliorates one or more symptoms associated with such disease or condition. For prophylactic use, the pharmaceutical composition will contain and provide a **"prophylactically effective"** amount of the protein biomolecule(s), which is an amount that is sufficient to result in the prevention of the development, recurrence, onset or progression of a disease or condition. The recipient subject is an animal, preferably a mammal including a non-primate (e.g., a cow, pig, horse, cat, dog, rat, or mouse), or a primate *(e.g.,* a chimpanzee, a monkey such as a cynomolgus monkey, and a human), and is more preferably a human.

### II. The Amorphous Stabilizing Compounds of the Pharmaceutical Compositions Of The Present Invention

The amorphous stabilizing compounds of the present invention are **"lyoprotectants"** (and as such serve to protect the protein biomolecule of the pharmaceutical composition from denaturation during freeze-drying and subsequent storage) and/or **"cryoprotectants"** (and as such serve to protect the protein biomolecule of the pharmaceutical composition from denaturation caused by freezing). An **"amorphous stabilizing"** compound is said to **"stabilize"** or **"protect"** a protein biomolecule of a pharmaceutical composition of the present invention, if it serves to preserve the structure and functionality of the protein biomolecule that is the active agent or component of the composition, relative to changes in such structure and functionality observed in the absence of such compound. A stabilizing compound is said to be an **"amorphous"** stabilizing compound or composition if it does not crystallize and stays uniformly distributed in the freeze concentrated matrix.

The **"protection"** provided to the protein biomolecule may be assessed using high performance size exclusion chromatography **("HPSEC"),** which is an industry standard technique for the detection and quantification of pharmaceutical protein aggregates (US Patent Publication No. 2015/0005475; Gabrielson, J.P. et al. (2006) "Quantitation Of Aggregate Levels In A Recombinant Humanized Monoclonal Antibody Formulation By Size-Exclusion Chromatography, Asymmetrical Flow Field Flow Fractionation, And Sedimentation Velocity," J. Pharm. Sci. 96(2):268-279; Liu, H. et al. (2009) "Analysis Of Reduced Monoclonal Antibodies Using Size Exclusion Chromatography Coupled With Mass Spectrometry," J. Amer. Soc. Mass Spectrom. 20:2258-2264; Mahler, H. C. et al. (2008) "Protein Aggregation: Pathways, Induction Factors And Analysis," J. Pharm. Sci. 98(9):2909-2934). Such protection permits the protein biomolecule to exhibit **"low to undetectable levels"** of fragmentation, *i.e.,* such that, in a sample of the pharmaceutical composition, more than 80%, 85%, 90% 95%, 98%, or 99% of the protein biomolecule migrates in a single peak as determined by HPSEC and/or **"low to undetectable levels"** of loss of the biological activity/ies associated, *i.e.,* such that, in a sample of the pharmaceutical composition, more than 80%, 85%, 90% 95%, 98%, or 99% of the protein biomolecule present exhibits its initial biological activity/ies as measured by HPSEC, and/or **low to undetectable levels"** of aggregation, *i.e.,* such that, in a sample of the pharmaceutical composition, no more than 5%, no more than 4%, no more than 3%, no more than 2%, no more than 1%, and most preferably no more than 0.5%, aggregation by weight protein as measured by HPSEC. The **"long-term"** stability provided by the pharmaceutical compositions of the present permit such compositions to be stored for more than 3 months, more than 6 months, more than 9 months, more than 1 year, more than 18 months, more than 2 years, or more than 30 months.

The preferred **"amorphous stabilizing compounds"** of the present invention may be composed of any amorphous stabilizing compound, or mixture of such compounds, that does not crystallize and stays uniformly distributed in freeze concentrated matrix. Suitable amorphous stabilizing compounds include sugars and organic molecules (e.g., budesonide, dextran DMSO glycerol, glucose, inulin, lactose, maltose, mannitol, PEG, piroxicam, PLGA, PVA sorbitol, sucrose, trehalose and urea) and amino acid molecules (e.g., alanine, arginine, glycine, lysine and/or proline), or derivatives and salts thereof, or mixtures thereof. Such amino acid molecules will preferably be L-amino acid molecules, but may be D-amino acid molecules or any combination of D- and L-amino acid molecules, including a racemic mixture thereof.

With respect to such amino acids molecules, the term **"derivatives and salts thereof"** denotes any pharmaceutically acceptable salt or amino acid derivative, such as those disclosed in REMINGTON: THE SCIENCE AND PRACTICE OF PHARMACY, 21th Edition, Gennaro, Ed., Mack Publishing Co., Easton, PA, 2005. Such derivatives include substituted amines, amino alcohols, aldehydes, lactones, esters, hydrates, *etc.* Exemplary derivatives of alanine include: 2-allyl-glycine, 2-aminobutyric acid, cis-amiclenomycin, adamanthane, *etc.* Exemplary derivatives of arginine include: 2-amino-3-guanidinopropionic acid, 2-amino-4-guanidinobutryric acid, 5-methyl-arginine, arginine methyl ester, arginine-O-tBu, canavanine, citrulline, c-γ-hydroxy arginine, homoarginine, N-tosyl-arginine, N_{ω}-nitro-arginine, thio-citrulline, *etc.* Exemplary derivatives of lysine include: diaminobutyric acid, 2,3-diaminopropanoic acid, (2s)-2,8-diaminoactanoic acid, ornithine, thialysine, *etc.* Exemplary derivatives of proline include: trans-1-acetyl-4-hydroxyproline, 3,4-dehydroproline, cis-3-hydroxyproline, cis-4-hydroxyproline, trans-3-hydroxyproline, trans-4-hydroxyproline, α-methylproline, pipecolic acid, *etc.*

Salts of such amino acids molecules and their derivatives include additional salts of such molecules such as those derived from an appropriate acid, *e.g.,* hydrochloric, sulphuric, phosphoric, maleic, fumaric, citric, tartaric, lactic, acetic or p-toluenesulphonic acid. Particularly preferred are hydrochloride salts.

With respect to such sugars, sucrose, trehalose, glucose, lactose, or sorbitol, or any mixture thereof, are particularly preferred, with the invention being illustrated below with respect to the use of sucrose as a preferred amorphous stabilizing compound.

Such stabilizing compounds can be used individually, or in combination, in a pharmaceutical composition of the present invention (e.g., such compositions may possess only a single stabilizing compound, any two stabilizing compounds, any three stabilizing compounds, any four stabilizing compounds, any five stabilizing compounds, or any combination of more than five of such stabilizing compounds).

### III. The Protein Biomolecules Of The Pharmaceutical Compositions Of The Present Invention

The stabilizing compositions of the present invention are particularly suitable for use in pharmaceutical compositions that contain high concentrations of one or more protein biomolecule(s) as their active agents or components. As used herein, the term **"high concentration"** denotes a concentration of the protein biomolecule(s) that is greater than 10 mg/mL, greater than 20 mg/mL, greater than 30 mg/mL, greater than 40 mg/mL, greater than 50 mg/mL, greater than 60 mg/mL, greater than 70 mg/mL, greater than 80 mg/mL, greater than 90 mg/mL, greater than 100 mg/mL, greater than 120 mg/mL, greater than 150 mg/mL, greater than 200 mg/mL, greater than 250 mg/mL, greater than 300 mg/mL, greater than 350 mg/mL, greater than 400 mg/mL, greater than 450 mg/mL, or greater than 500 mg/mL.

Without limitation, the **"protein biomolecules"** contained in such pharmaceutical compositions may be any kind of protein molecule, including single polypeptide chain proteins or multiple polypeptide chain proteins. As used herein the term protein biomolecule does not connote that the molecule is of any particular size and is intended to include protein biomolecules that comprise fewer than 5, fewer than 10, fewer than 20 fewer than 30, fewer than 40 or fewer than 50 amino acid residues, as well as protein biomolecules that comprise more than 50, more than 100, more than 200 more than 300, more than 400, or more than 500 amino acid residues.

Examples of protein biomolecules that may be present in the pharmaceutical compositions of the present invention are provided in **Tables 1** and **2,** and include antibody or antibody-based immunotherapeutics (for example, palivizumab which is directed to an epitope in the A antigenic site of the F protein of respiratory syncytial virus (RSV) (SYNAGIS^{®}; US Patents No. 8,460,663 and 8,986,686), antibody directed against angiopoietin-2 (US Patents No. 8,507,656 and 8,834,880); antibody directed against Delta-like Protein Precursor 4 (DLL4) (US Patent No. 8,663,636; US Patent Publication No. 2015/0005475; PCT Publication No. WO 2013/113898); antibody directed against Platelet-Derived Growth Factor-α (PDGRF-α) (US Patent No. 8,697,664); antibody directed against alpha-V-beta-6 integrin (αVβ6) (US Patent No. 8,894,998; antibody directed against Growth and Differentiation Factor (GDF-8) (US Patent No. 8,697,664), enzymes, hormones and factors, and antigenic proteins for use in vaccines (for example, insulin, erythropoietin, growth hormone, *etc.).*

| **Table 1** | | |
|---|---|---|
| **Antibody and Immunotherapeutic Molecules** | | |
| **Antibody Name** | **Disease-Associated Antigen** | **Therapeutic Target Application** |
| 3F8 | Gd2 | Neuroblastoma |
| 8H9 | B7-H3 | Neuroblastoma, Sarcoma, Metastatic Brain Cancers |
| Abagovomab | CA-125 | Ovarian Cancer |
| Abciximab | CD41 | Platelet Aggregation Inhibitor |
| Actoxumab | *Clostridium Difficile* | *Clostridium Difficile* Infection |
| Adalimumab | TNF-A | Rheumatoid Arthritis, Crohn's Disease, Plaque Psoriasis, Psoriatic Arthritis, Ankylosing Spondylitis, Juvenile Idiopathic Arthritis, Hemolytic Disease Of The Newborn |
| Adecatumumab | Epcam | Prostate And Breast Cancer |
| Aducanumab | Beta-Amyloid | Alzheimer's Disease |
| Afelimomab | TNF-A | Sepsis |
| Afutuzumab | CD20 | Lymphoma |
| Alacizumab | VEGFR2 | Cancer |
| Ald518 | Il-6 | Rheumatoid Arthritis |
| Alemtuzumab | CD52 | Multiple Sclerosis |
| Alirocumab | NARP-1 | Hypercholesterolemia |
| Altumomab | CEA | Colorectal Cancer |
| Amatuximab | Mesothelin | Cancer |
| Anatumomab Mafenatox | TAG-72 | Non-Small Cell Lung Carcinoma |
| Anifrolumab | Interferon A/B Receptor | Systemic Lupus Erythematosus |
| Anrukinzumab | IL-13 | Cancer |
| Apolizumab | HLA-DR | Hematological Cancers |
| Arcitumomab | CEA | Gastrointestinal Cancer |
| Aselizumab | L-Selectin (CD62L) | Severely Injured Patients |
| Atinumab | RTN4 | Cancer |
| Atlizumab | IL-6 Receptor | Rheumatoid Arthritis |
| Atorolimumab | Rhesus Factor | Hemolytic Disease Of The Newborn |
| Bapineuzumab | Beta-Amyloid | Alzheimer's Disease |
| Basiliximab | CD25 | Prevention Of Organ Transplant Rejections |
| Bavituximab | Phosphatidylserine | Cancer, Viral Infections |
| Bectumomab | CD22 | Non-Hodgkin's Lymphoma (Detection) |
| Belimumab | BAFF | Non-Hodgkin Lymphoma |
| Benralizumab | CD125 | Asthma |
| Bertilimumab | CCL11 (Eotaxin-1) | Severe Allergic Disorders |
| Besilesomab | CEA-Related Antigen | Inflammatory Lesions And Metastases (Detection) |
| Bevacizumab | VEGF-A | Metastatic Cancer, Retinopathy Of Prematurity |
| Bezlotoxumab | *Clostridium difficile* | *Clostridium difficile* Infection |
| Biciromab | Fibrin II, Beta Chain | Thromboembolism (Diagnosis) |
| Bimagrumab | ACVR2B | Myostatin Inhibitor |
| Bivatuzumab | CD44 V6 | Squamous Cell Carcinoma |
| Blinatumomab | CD19 | Cancer |
| Blosozumab | SOST | Osteoporosis |
| Brentuximab | CD30 (TNFRSF8) | Hematologic Cancers |
| Briakinumab | IL-12, IL-23 | Psoriasis, Rheumatoid Arthritis, Inflammatory Bowel Diseases, Multiple Sclerosis |
| Brodalumab | IL-17 | Inflammatory Diseases |
| Canakinumab | IL-1 | Rheumatoid Arthritis |
| Cantuzumab | MUC1 | Cancers |
| Cantuzumab Mertansine | Mucin Canag | Colorectal Cancer |
| Caplacizumab | VWF | Cancers |
| Capromab | Prostatic Carcinoma Cells | Prostate Cancer (Detection) |
| Carlumab | MCP-1 | Oncology/Immune Indications |
| Catumaxomab | Epcam, CD3 | Ovarian Cancer, Malignant Ascites, Gastric Cancer |
| Cc49 | Tag-72 | Tumor Detection |
| Certolizumab | TNF-A | Crohn's Disease |
| Cetuximab | EGFR | Metastatic Colorectal Cancer And Head And Neck Cancer |
| Ch.14.18 | Undetermined | Neuroblastoma |
| Citatuzumab | Epcam | Ovarian Cancer And Other Solid Tumors |
| Cixutumumab | IGF-1 Receptor | Solid Tumors |
| Clazakizumab | *Oryctolagus Cuniculus* | Rheumatoid Arthritis |
| Clivatuzumab | MUC1 | Pancreatic Cancer |
| Conatumumab | TRAIL-R2 | Cancer |
| Concizumab | TFPI | Bleeding |
| Cr6261 | Influenza A Hemagglutinin | Infectious Disease/Influenza A |
| Crenezumab | 1-40-B-Amyloid | Alzheimer's Disease |
| Dacetuzumab | CD40 | Hematologic Cancers |
| Daclizumab | CD25 | Prevention Of Organ Transplant Rejections |
| Dalotuzumab | Insulin-Like Growth Factor I Receptor | Cancer |
| Daratumumab | CD38 | Cancer |
| Demcizumab | DLL4 | Cancer |
| Denosumab | RANKL | Osteoporosis, Bone Metastases |
| Detumomab | B-Lymphoma Cell | Lymphoma |
| Dorlimomab Aritox | Undetermined | Cancer |
| Drozitumab | DR5 | Cancer |
| Duligotumab | HER3 | Cancer |
| Dupilumab | IL4 | Atopic Diseases |
| Dusigitumab | ILGF2 | Cancer |
| Ecromeximab | GD3 Ganglioside | Malignant Melanoma |
| Eculizumab | C5 | Paroxysmal Nocturnal Hemoglobinuria |
| Edobacomab | Endotoxin | Sepsis Caused By Gram-Negative Bacteria |
| Edrecolomab | Epcam | Colorectal Carcinoma |
| Efalizumab | LFA-1 (CD11a) | Psoriasis (Blocks T Cell Migration) |
| Efungumab | Hsp90 | Invasive Candida Infection |
| Eldelumab | Interferon-Gamma-Induced Protein | Crohn's Disease, Ulcerative Colitis |
| Elotuzumab | SLAMF7 | Multiple Myeloma |
| Elsilimomab | IL-6 | Cancer |
| Enavatuzumab | TWEAK Receptor | Cancer |
| Enlimomab | ICAM-1 (CD54) | Cancer |
| Enokizumab | IL9 | Asthma |
| Enoticumab | DLL4 | Cancer |
| Ensituximab | 5AC | Cancer |
| Epitumomab Cituxetan | Episialin | Cancer |
| Epratuzumab | CD22 | Cancer, SLE |
| Erlizumab | ITGB2 (CD18) | Heart Attack, Stroke, Traumatic Shock |
| Ertumaxomab | HER2/Neu, CD3 | Breast Cancer |
| Etaracizumab | Integrin Aᵥβ₃ | Melanoma, Prostate Cancer, Ovarian Cancer |
| Etrolizumab | Integrin A₇ B₇ | Inflammatory Bowel Disease |
| Evolocumab | PCSK9 | Hypocholesterolemia |
| Exbivirumab | Hepatitis B Surface Antigen | Hepatitis B |
| Fanolesomab | CD15 | Appendicitis (Diagnosis) |
| Faralimomab | Interferon Receptor | Cancer |
| Farletuzumab | Folate Receptor 1 | Ovarian Cancer |
| Fasinumab^{[51]} | HNGF | Cancer |
| Fbta05 | CD20 | Chronic Lymphocytic Leukaemia |
| Felvizumab | Respiratory Syncytial Virus | Respiratory Syncytial Virus Infection |
| Fezakinumab | IL-22 | Rheumatoid Arthritis, Psoriasis |
| Ficlatuzumab | HGF | Cancer |
| Figitumumab | IGF-1 Receptor | Adrenocortical Carcinoma, Non-Small Cell Lung Carcinoma |
| Flanvotumab | TYRP1 (Glycoprotein 75) | Melanoma |
| Fontolizumab | IFN-γ | Crohn's Disease |
| Foravirumab | Rabies Virus Glycoprotein | Rabies (Prophylaxis) |
| Fresolimumab | TGF-B | Idiopathic Pulmonary Fibrosis, Focal Segmental Glomerulosclerosis, Cancer |
| Fulranumab | NGF | Pain |
| Futuximab | EGFR | Cancer |
| Galiximab | CD80 | B Cell Lymphoma |
| Ganitumab | IGF-I | Cancer |
| Gantenerumab | Beta-Amyloid | Alzheimer's Disease |
| Gavilimomab | CD147 (Basigin) | Graft-Versus-Host Disease |
| Gemtuzumab Ozogamicin | CD33 | Acute Myelogenous Leukemia |
| Gevokizumab | IL-1β | Diabetes |
| Girentuximab | Carbonic Anhydrase 9 (CA-IX) | Clear Cell Renal Cell Carcinoma^{[64]} |
| Glembatumumab Vedotin | GPNMB | Melanoma, Breast Cancer |
| Golimumab | TNF-A | Rheumatoid Arthritis, Psoriatic Arthritis, Ankylosing Spondylitis |
| Gomiliximab | CD23 (Ige Receptor) | Allergic Asthma |
| Guselkumab | IL13 | Psoriasis |
| Ibritumomab Tiuxetan | CD20 | Non-Hodgkin's Lymphoma |
| Icrucumab | VEGFR-1 | Cancer |
| Igovomab | CA-125 | Ovarian Cancer (Diagnosis) |
| Imab362 | Cldn18.2 | Gastrointestinal Adenocarcinomas And Pancreatic Tumor |
| Imgatuzumab | EGFR | Cancer |
| Inclacumab | Selectin P | Cancer |
| Indatuximab Ravtansine | SDC1 | Cancer |
| Infliximab | TNF-A | Rheumatoid Arthritis, Ankylosing Spondylitis, Psoriatic Arthritis, Psoriasis, Crohn's Disease, Ulcerative Colitis |
| Inolimomab | CD25 (A Chain Of IL-2 Receptor) | Graft-Versus-Host Disease |
| Inotuzumab Ozogamicin | CD22 | Cancer |
| Intetumumab | CD51 | Solid Tumors (Prostate Cancer, Melanoma) |
| Ipilimumab | CD152 | Melanoma |
| Iratumumab | CD30 (TNFRSF8) | Hodgkin's Lymphoma |
| Itolizumab | CD6 | Cancer |
| Ixekizumab | IL-17A | Autoimmune Diseases |
| Keliximab | CD4 | Chronic Asthma |
| Labetuzumab | CEA | Colorectal Cancer |
| Lambrolizumab | PDCD1 | Antineoplastic Agent |
| Lampalizumab | CFD | Cancer |
| Lebrikizumab | IL-13 | Asthma |
| Lemalesomab | NCA-90 (Granulocyte Antigen) | Diagnostic Agent |
| Lerdelimumab | TGF Beta 2 | Reduction Of Scarring After Glaucoma Surgery |
| Lexatumumab | TRAIL-R2 | Cancer |
| Libivirumab | Hepatitis B Surface Antigen | Hepatitis B |
| Ligelizumab | IGHE | Cancer |
| Lintuzumab | CD33 | Cancer |
| Lirilumab | KIR2D | Cancer |
| Lodelcizumab | PCSK9 | Hypercholesterolemia |
| Lorvotuzumab | CD56 | Cancer |
| Lucatumumab | CD40 | Multiple Myeloma, Non-Hodgkin's Lymphoma, Hodgkin's Lymphoma |
| Lumiliximab | CD23 | Chronic Lymphocytic Leukemia |
| Mapatumumab | TRAIL-R1 | Cancer |
| Margetuximab | Ch4d5 | Cancer |
| Matuzumab | EGFR | Colorectal, Lung And Stomach Cancer |
| Mavrilimumab | GMCSF Receptor A-Chain | Rheumatoid Arthritis |
| Mepolizumab | IL-5 | Asthma And White Blood Cell Diseases |
| Metelimumab | TGF Beta 1 | Systemic Scleroderma |
| Milatuzumab | CD74 | Multiple Myeloma And Other Hematological Malignancies |
| Minretumomab | TAG-72 | Cancer |
| Mitumomab | GD3 Ganglioside | Small Cell Lung Carcinoma |
| Mogamulizumab | CCR4 | Cancer |
| Morolimumab | Rhesus Factor | Cancer |
| Motavizumab | Respiratory Syncytial Virus | Respiratory Syncytial Virus (Prevention) |
| Moxetumomab Pasudotox | CD22 | Cancer |
| Muromonab-CD3 | CD3 | Prevention Of Organ Transplant Rejections |
| Nacolomab Tafenatox | C242 Antigen | Colorectal Cancer |
| Namilumab | CSF2 | Cancer |
| Naptumomab Estafenatox | 5T4 | Non-Small Cell Lung Carcinoma, Renal Cell Carcinoma |
| Narnatumab | RON | Cancer |
| Natalizumab | Integrin A4 | Multiple Sclerosis, Crohn's Disease |
| Nebacumab | Endotoxin | Sepsis |
| Necitumumab | EGFR | Non-Small Cell Lung Carcinoma |
| Nerelimomab | TNF-A | Cancer |
| Nesvacumab | Angiopoietin 2 | Cancer |
| Nimotuzumab | EGFR | Squamous Cell Carcinoma, Head And Neck Cancer, Nasopharyngeal Cancer, Glioma |
| Nivolumab | PD-1 | Cancer |
| Nofetumomab Merpentan | Undetermined | Cancer |
| Ocaratuzumab | CD20 | Cancer |
| Ocrelizumab | CD20 | Rheumatoid Arthritis, Lupus Erythematosus |
| Odulimomab | LFA-1 (CD11a) | Prevention Of Organ Transplant Rejections, Immunological Diseases |
| Ofatumumab | CD20 | Chronic Lymphocytic Leukemia |
| Olaratumab | PDGF-R A | Cancer |
| Olokizumab | IL6 | Cancer |
| Onartuzumab | Human Scatter Factor Receptor Kinase | Cancer |
| Ontuxizumab | TEM1 | Cancer |
| Oportuzumab Monatox | Epcam | Cancer |
| Oregovomab | CA-125 | Ovarian Cancer |
| Orticumab | Oxldl | Cancer |
| Otlertuzumab | CD37 | Cancer |
| Oxelumab | OX-40 | Asthma |
| Ozanezumab | NOGO-A | ALS And Multiple Sclerosis |
| Ozoralizumab | TNF-A | Inflammation |
| Pagibaximab | Lipoteichoic Acid | Sepsis (*Staphylococcus*) |
| Palivizumab | F Protein Of Respiratory Syncytial Virus | Respiratory Syncytial Virus (Prevention) |
| Panitumumab | EGFR | Colorectal Cancer |
| Pankomab | Tumor Specific Glycosylation Of MUC1 | Ovarian Cancer |
| Panobacumab | Pseudomonas Aeruginosa | *Pseudomonas Aeruginosa* Infection |
| Parsatuzumab | EGFL7 | Cancer |
| Pascolizumab | IL-4 | Asthma |
| Pateclizumab | LTA | TNF |
| Patritumab | HER3 | Cancer |
| Pembrolizumab | PD-1 | Cancer |
| Pemtumomab | MUC1 | Cancer |
| Perakizumab | IL17A | Arthritis |
| Pertuzumab | HER2/Neu | Cancer |
| Pexelizumab | C5 | Reduction Of Side-Effects Of Cardiac Surgery |
| Pidilizumab | PD-1 | Cancer And Infectious Diseases |
| Pinatuzumab Vedotin | CD22 | Cancer |
| Pintumomab | Adenocarcinoma Antigen | Adenocarcinoma |
| Placulumab | Human TNF | Cancer |
| Polatuzumab Vedotin | CD79B | Cancer |
| Ponezumab | Human Beta-Amyloid | Alzheimer's Disease |
| Pritoxaximab | *E. Coli* Shiga Toxin Type-1 | Cancer |
| Pritumumab | Vimentin | Brain Cancer |
| Pro 140 | Ccr5 | HIV Infection |
| Quilizumab | IGHE | Cancer |
| Racotumomab | N-Glycolylneuraminic Acid | Cancer |
| Radretumab | Fibronectin Extra Domain-B | Cancer |
| Rafivirumab | Rabies Virus Glycoprotein | Rabies (Prophylaxis) |
| Ramucirumab | VEGFR2 | Solid Tumors |
| Ranibizumab | VEGF-A | Macular Degeneration (Wet Form) |
| Raxibacumab | Anthrax Toxin, Protective Antigen | Anthrax (Prophylaxis And Treatment) |
| Regavirumab | Cytomegalovirus Glycoprotein B | Cytomegalovirus Infection |
| Reslizumab | IL-5 | Inflammations Of The Airways, Skin And Gastrointestinal Tract |
| Rilotumumab | HGF | Solid Tumors |
| Rituximab | CD20 | Lymphomas, Leukemias, Some Autoimmune Disorders |
| Robatumumab | IGF-1 Receptor | Cancer |
| Roledumab | RHD | Cancer |
| Romosozumab | Sclerostin | Osteoporosis |
| Rontalizumab | IFN-α | Systemic Lupus Erythematosus |
| Rovelizumab | CD11, CD18 | Haemorrhagic Shock |
| Ruplizumab | CD154 (CD40L) | Rheumatic Diseases |
| Samalizumab | CD200 | Cancer |
| Sarilumab | IL6 | Rheumatoid Arthritis, Ankylosing Spondylitis |
| Satumomab Pendetide | TAG-72 | Cancer |
| Secukinumab | IL-17A | Uveitis, Rheumatoid Arthritis Psoriasis |
| Seribantumab | ERBB3 | Cancer |
| Setoxaximab | *E. Coli* Shiga Toxin Type-1 | Cancer |
| Sevirumab | Cytomegalovirus | Cytomegalovirus Infection |
| Sgn-CD19a | CD19 | Acute Lymphoblastic Leukemia And B Cell Non-Hodgkin Lymphoma |
| Sgn-CD33a | CD33 | Acute Myeloid Leukemia |
| Sibrotuzumab | FAP | Cancer |
| Sifalimumab | IFN-A | SLE, Dermatomyositis, Polymyositis |
| Siltuximab | IL-6 | Cancer |
| Simtuzumab | LOXL2 | Fibrosis |
| Siplizumab | CD2 | Psoriasis, Graft-Versus-Host Disease (Prevention) |
| Sirukumab | IL-6 | Rheumatoid Arthritis |
| Solanezumab | Beta-Amyloid | Alzheimer's Disease |
| Solitomab | Epcam | Cancer |
| Sonepcizumab | Sphingosine-1-Phosphate | Choroidal And Retinal Neovascularization |
| Sontuzumab | Episialin | Cancer |
| Stamulumab | Myostatin | Muscular Dystrophy |
| Sulesomab | NCA-90 (Granulocyte Antigen) | Osteomyelitis |
| Suvizumab | HIV-1 | Viral Infections |
| Tabalumab | BAFF | B Cell Cancers |
| Tacatuzumab Tetraxetan | Alpha-Fetoprotein | Cancer |
| Tadocizumab | Integrin AIIBβ3 | Percutaneous Coronary Intervention |
| Tanezumab | NGF | Pain |
| Taplitumomab Paptox | CD19 | Cancer |
| Tefibazumab | Clumping Factor A | *Staphylococcus Aureus* Infection |
| Telimomab | Undetermined | Cancer |
| Tenatumomab | Tenascin C | Cancer |
| Teneliximab | CD40 | Cancer |
| Teprotumumab | CD221 | Hematologic Tumors |
| Ticilimumab | CTLA-4 | Cancer |
| Tigatuzumab | TRAIL-R2 | Cancer |
| Tildrakizumab | IL23 | Immunologically Mediated Inflammatory Disorders |
| Tnx-650 | Il-13 | Hodgkin's Lymphoma |
| Tocilizumab | IL-6 Receptor | Rheumatoid Arthritis |
| Toralizumab | CD154 (CD40L) | Rheumatoid Arthritis, Lupus Nephritis |
| Tositumomab | CD20 | Follicular Lymphoma |
| Tovetumab | CD 140a | Cancer |
| Tralokinumab | IL-13 | Asthma |
| Trastuzumab | HER2/Neu | Breast Cancer |
| Trbs07 | Gd2 | Melanoma |
| Tremelimumab | CTLA-4 | Cancer |
| Tucotuzumab Celmoleukin | Epcam | Cancer |
| Tuvirumab | Hepatitis B Virus | Chronic Hepatitis B |
| Ublituximab | MS4A1 | Cancer |
| Urelumab | 4-1BB | Cancer |
| Urtoxazumab | Escherichia Coli | Diarrhoea Caused By *E. Coli* |
| Ustekinumab | IL-12, IL-23 | Multiple Sclerosis, Psoriasis, Psoriatic Arthritis |
| Vantictumab | Frizzled Receptor | Cancer |
| Vapaliximab | AOC3 (VAP-1) | Cancer |
| Vatelizumab | ITGA2 | Cancer |
| Vedolizumab | Integrin A4β7 | Crohn's Disease, Ulcerative Colitis |
| Veltuzumab | CD20 | Non-Hodgkin's Lymphoma |
| Vepalimomab | AOC3 (VAP-1) | Inflammation |
| Vesencumab | NRP1 | Cancer |
| Volociximab | Integrin A5β1 | Solid Tumors |
| Vorsetuzumab | CD70 | Cancer |
| Votumumab | Tumor Antigen CTAA16.88 | Colorectal Tumors |
| Zalutumumab | EGFR | Squamous Cell Carcinoma Of The Head And Neck |
| Zatuximab | HER1 | Cancer |
| Ziralimumab | CD147 | Cancer |
| Zolimomab Aritox | CD5 | Systemic Lupus Erythematosus, Graft-Versus-Host Disease |

| **Table 2** |
|---|
| **Hormones / Factors** |
| Alpha-Glactosidase A |
| Alpha-L-Iduronidase |
| Dornase Alfa |
| Erythropoietin |
| Factor VIII |
| Follicle-Stimulating Hormone |
| Glucocerebrosidase |
| Granulocyte Colony-Stimulating Factor (G-CSF) |
| Growth Hormone |
| Insulin |
| Insulin-Like Growth Factor 1 (IGF-1) |
| Interferon-B-1a |
| Interferon-β-1b |
| N-Acetylgalactosamine-4-Sulfatase |
| Tissue Plasminogen Activator (TPA) |

### IV. Formulation of the Pharmaceutical Compositions of the Present Invention

The pharmaceutical compositions of the present invention will typically be formulated, at least initially, as an aqueous liquid, but are most preferably then suitable for lyophilization. A pharmaceutical composition of the present invention subsequent to such lyophilization is referred to herein as a **"lyophilisate."**

The liquid formulations of the pharmaceutical compositions of the present invention preferably comprise a suitable sterile aqueous carrier, a high concentration (as defined above) of the protein biomolecule, a buffer, and a stabilizing compound of the present invention. Optionally, such liquid formulations of the pharmaceutical compositions of the present invention may contain additional components, for example, a pharmaceutically acceptable, non-toxic excipient, buffer or detergent.

Examples of suitable sterile aqueous carriers which may be employed in the pharmaceutical compositions of the present invention include water, saline, phosphate buffered saline, ethanol, dextrose solutions, and water/polyol solutions (such as glycerol, propylene glycol, polyethylene glycol, and the like).

Any suitable buffer may be employed in accordance with the present invention. It is preferred to employ a buffer capable of buffering the liquid within a range of from about 3 to about 11, from about 4 to about 9, from about 5 to about 8, from about 5 to about 7.5, preferably at a pH of 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0. Suitable buffers include potassium phosphate, sodium phosphate, sodium acetate, histidine, imidazole, sodium citrate, sodium succinate, ammonium bicarbonate and carbonate. Generally, buffers are used at molarities from about 1 mM to about 2 M, from about 2 mM to about 1 M being preferred, and from about 10 mM to about 0.5 M being especially preferred, and 25 to 50 mM being particularly preferred.

In one embodiment, the buffer is histidine / histidine-HCl and is included in the liquid formulations of the invention in a range of from about 1 mM to about 100 mM, about 10 mM to about 50 mM, about 20 mM to about 30 mM, or about 23 mM to about 27 mM, and is most preferably about 25 mM. Histidine can be in the form of L-histidine, D-histidine, or a mixture thereof, but L-histidine is the most preferable. Histidine can be also in the form of a hydrate, or a pharmaceutically acceptable salt, such as hydrochloride *(e.g.,* a monohydrochloride or a dihydrochloride), hydrobromide, sulfate, acetate, *etc.* The purity of the histidine should be at least 98%, preferably at least 99%, and most preferably at least 99.5%.

The concentration of the amorphous stabilizing compound(s) that is/are included in the pharmaceutical composition of the present invention preferably ranges from about 0.1% (weight/volume (w/v)) to about 8.5% (w/v), more preferably from about 0.1% (w/v) to about 2% (w/v) or from about 0.3% (w/v) to about 1.5% (w/v) or from about 0.5% (w/v) to about 2.5% (w/v)). Particularly preferred are amorphous stabilizing compositions of 0.5-1% sugar (w/v) (especially sucrose).

Preferably, pharmaceutical compositions that contain 50 mg/mL or less of a protein biomolecule will comprise about 0.1%, 0.2%, 0.3%, 0.4%, 0.5%, 1%, 1.5%, 2% or 2.5% amorphous stabilizing compound (w/v) *(i.e.,* the pharmaceutical composition will have a protein biomolecule to amorphous stabilizing compound ratio of 1:0.02 (w/w), 1:0.04 (w/w), 1:0.06 (w/w), 1:0.08 (w/w) or 1:0.1 (w/w), 1:0.2 (w/w), 1:0.3 (w/w), 1:0.4 (w/w), and 1:0.5 (w/w), respectively for a protein biomolecule present at a concentration of 50 mg/mL.

Preferably, pharmaceutical compositions that contain more than 50 mg/mL, and more preferably, 100 mg/mL or more, of a protein biomolecule will comprise about 1%, greater than about 1%, 1.5%, 2%, 2.5%, 3%, 3.5%, 4%, 4.5%, 5%, 5.5%, 6%, 6.5%, 7% 7.5%, 8% or 8.5% amorphous stabilizing compound (w/v) *(i.e.,* the pharmaceutical composition will have a protein biomolecule to amorphous stabilizing compound ratio of 1:0.1 (w/w), 1:0.15 (w/w), 1:0.2 (w/w), 1:0.25 (w/w) or 1:0.3 (w/w), 1:0.35 (w/w), 1:0.4 (w/w), 1:0.45 (w/w), and 1:0.5 (w/w), 1:0.55 (w/w), 1:0.6 (w/w), 1:0.65 (w/w), 1:0.7 (w/w), 1:0.75 (w/w), 1:0.8 (w/w), 1:0.85 (w/w), respectively for a protein biomolecule present at a concentration of 100 mg/mL).

Particularly preferred pharmaceutical compositions that contain 50 mg/mL or less of a protein biomolecule will additionally contain 0.5% sucrose (w/v) *(i.e.,* the pharmaceutical composition will have a protein biomolecule to sugar ratio of 1:0.1 (w/w)), or 1% sucrose (w/v) *(i.e.,* the pharmaceutical composition will have a protein biomolecule to sugar ratio of 1:0.2 (w/w)). Particularly preferred pharmaceutical compositions that contain 100 mg/mL or more of a protein biomolecule will additionally contain 1% sucrose (w/v) *(i.e.,* the pharmaceutical composition will have a protein biomolecule to sugar ratio of 1:0.1 (w/w)).

Polysorbate-80 **("PS-80")** is a preferred non-ionic surfactant and emulsifier of the present invention, however, other suitable non-ionic surfactants and emulsifiers (e.g., Tween-20^{®}, Tween-80^{®}, Poloxamer, sodium dodecyl sulfate, *etc.)* may be alternatively or additionally employed.

The liquid formulation can be lyophilized to further stabilize the protein biomolecule. Any suitable lyophilization apparatus and regimen may be employed, however, it is preferred to accomplish such lyophilization as shown in **Table 4.**

Particularly subsequent to reconstitution after such lyophilization, liquid formulations of the pharmaceutical compositions of the present invention may additionally contain non-aqueous carriers, such as mineral oil or vegetable oil *(e.g.,* olive oil, corn oil, peanut oil, cottonseed oil, and sesame oil), carboxymethyl cellulose colloidal solutions, tragacanth gum and injectable organic esters, such as ethyl oleate.

The invention provides methods of treatment, prophylaxis, and amelioration of a disease, disorder or condition or one or more symptoms thereof by administrating to a subject of an effective amount of liquid formulations of the invention, either as initially formulated or subsequent to reconstitution of a lyophilisate. Thus, the invention provides a method of treating a disease or disorder by administering such a pharmaceutical composition (containing, for example, an antibody of Table 1, or a derivative or fragment of such an antibody, or a hormone or factor of Table 2, or a derivative thereof) to a recipient patient in need of such treatment. The pharmaceutical compositions of the present invention thus have use in medicine and in medical care.

Various delivery systems are known and can be used to administer such liquid compositions, including, but not limited to, parenteral administration (e.g., intradermal, intramuscular, intraperitoneal intravenous and subcutaneous), epidural administration, topical administration, pulmonary administration, and mucosal administration (e.g., intranasal and oral mutes). In a specific embodiment, liquid formulations of the present invention are administered intramuscularly, intravenously or subcutaneously. The formulations may be administered by any convenient mute, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, pulmonary administration can be employed, *e.g.,* by use of an inhaler or nebulizer.

The invention also provides that the initially formulated liquid pharmaceutical composition may be packaged in a hermetically sealed container such as an ampoule, vial, syringe, cartridge or sachette indicating the quantity of the protein biomolecule contained therein. Preferably, such initially formulated liquid pharmaceutical compositions are lyophilized while within such ampoules, vials, syringes, cartridges or sachettes, and the ampoule, vial, syringe, cartridge or sachette indicates the amount of carrier to be added in order to reconstitute the lyophilisate to contain the desired high concentration of the protein biomolecule.

The amount of the liquid formulations of the present invention which will be effective for therapeutic or prophylactic use will be determined by the treating physician, and will depend on factors such as the age and weight of the intended recipient patient, the disease or condition being treated, *etc.*

The precise dose to be employed in the formulation will also depend on the route of administration, the disease or condition to be treated, the particular protein biomolecule of the pharmaceutical composition, and should be decided according to the judgment of the practitioner and each subject's circumstances. Exemplary doses include 30 mg/kg or less, 15 mg/kg or less, 5 mg/kg or less, 3 mg/kg or less, 1 mg/kg or less or 0.5 mg/kg or less.

### EXAMPLES

The following examples illustrate the compositions of the present invention and their properties. The examples are intended to illustrate, but in no way limit, the scope of the invention.

### Example 1

### Materials & Methods

Stability studies were performed to evaluate the effect of sugar concentration on the stability of pharmaceutical compositions that contain high concentrations of protein biomolecules. The studies employed pharmaceutical compositions that contained, as exemplary protein biomolecules, either a Tenascin-3-Human Serum Albumin (Tn3-HSA) fusion protein (at 50 mg/mL) or a humanized IgG1 antibody (at 50 mg/mL or 100 mg/mL). For such studies, 1.1 mL of various pharmaceutical compositions **(Table** 3) was introduced into 3 cc vials. The vials were stoppered with 13 mm single vent lyophilization stoppers. The vials were then lyophilized using a lyophilization cycle, as described in **Table 4.**

| **Table 3** | | | | |
|---|---|---|---|---|
| **Pharmaceutical Compositions Evaluated For Protein : Sugar Ratio Study** | | | | |
| **Protein Biomolecule** | **Protein Concentration** | **Pharmaceutical Composition** | | |
| | | **Histidine, Excipient and pH** | **Percent Sucrose Concentration. (w/v)** | **Protein Biomolecule to Sugar Ratio (w/w)** |
| Humanized IgG1 Antibody | 50 mg/mL | 25 mM | 0 % | 1:0 |
| | | Histidine / Histidine-HCl | 0.5 % | 1 : 0.1 |
| | | | 1 % | 1: 0.2 |
| | | 0.02% PS-80 | 2.5 % | 1 : 0.5 1 : 1 |
| | | pH 6.0 | 5 % | |
| | 100 mg/mL | | 0.1 % | 1 : 0.01 |
| | | 25 mM | 1 % | 1 : 0.1 |
| | | Histidine / Histidine-HCl | 2 % | 1 : 0.2 |
| | | | 3 % | 1 : 0.3 |
| | | 0.02% PS-80 | 5 % | 1 : 0.5 |
| | | pH 6.0 | 8.5 % | 1 : 0.85 |
| | | | 10 % | 1: 1 |
| Tn3-HSA Fusion Protein | 50 mg/mL | 10 mM | 1 % | 1: 0.2 |
| | | Phosphate | 2.5 % | 1:0.5 |
| | | 0.02% PS-80 | 5 % | 1:1 |
| | | pH 7.4 | | |

For the controlled freeze-thaw study performed for the humanized IgG1 antibody, sucrose concentrations of 0 to 3% (w/v) were evaluated for formulations with protein concentrations of 100 mg/mL, whereas sucrose concentrations from 0 to 4% (w/v) were evaluated for formulations with protein concentrations of 50 mg/mL.

All lyophilization runs were performed with at least 4 thermocouple vials (two center and 2 edge vials). Lyophilization cycles were performed on a Virtis lyophilizer having a radiation shield on its door.

| **Table 4** | | | | |
|---|---|---|---|---|
| **Lyophilization Conditions** | | | | |
| **Lyophilization Step** | **Ramp Rate (°C/min)** | **Set point (°C)** | **Pressure (mTorr)** | **Duration (min)** |
| Loading | NA | 22 | NA | NA |
| Freezing | 0.5 | 5 | | 30 |
| Freezing | 0.5 | -40 | | 120 |
| Annealing | 0.5 | -16 | | 120 |
| Freezing | 0.5 | -40 | | 120 |
| Primary Drying | 0.3 | -25 | 100 | Drying time up to 8640 mins |
| Secondary Drying | 0.5 | 40 | 100 | 360 |
| Unloading | 0.5 | 5 | NA | NA |
| NA: Not Applicable | | | | |

The end point of lyophilization was determined using a Pirani vacuum gauge (see, *e.g.,* Patel, S.M. et al. (2009) "Determination of End Point of Primary Drying in Freeze-Drying Process Control," AAPS Pharm. Sci. Tech. 11(1):73-84). Such a gauge works on the principle of measuring the thermal conductivity of the gas in the drying chamber (Nail, S.L. et al. (1992) "Methodology For In-Process Determination Of Residual Water In Freeze-Dried Products," Dev. Biol. Stand. 74:137-151; Biol. Prod. Freeze-Drying Formulation). After completion of the lyophilization cycles, vials were vacuum stoppered and removed from the lyophilizer. The vials were then capped with 13 mm aluminum Flip-Off overseals.

**High Performance Size-Exclusion Chromatography (HPSEC)** - HPSEC samples were diluted in 10 mg/mL phosphate buffered saline prior to HPSEC. The samples were injected onto a TSKgel G3000SWXL column, eluted isocratically with phosphate buffer containing sodium sulfate and sodium azide. The eluted protein is detected using UV absorbance at 280 nm and the results are reported as the area percent of the product monomer peak. Peaks eluting earlier than the monomer are recorded as percent aggregate and peaks eluting after the monomer are recorded as percent fragment/other.

**Reconstitution Procedure** - Prior to use, and generally within 6 hours prior to use, sterile water is injected into the lyophilization vial, which is then gently swirled to effect reconstitution with minimal foaming. Reconstitution time was determined at major time-points of the stability studies. The vials were reconstituted with water. Water was directed to the vial wall and vials were swirled intermittently. When all solids were completely dissolved, the reconstitution time was recorded. Reconstitution time was within 25 to 40 mins for the 100 mg/ml formulations and ≤ 10 mins for the 50 mg/mL formulations. No significant trend in reconstitution time was observed for varying levels of sugars of compositions of the humanized IgG1 antibody (at 50 mg/mL or 100 mg/mL) or the Tn3-HSA fusion protein (at 50 mg/mL).

| **Table 5** | | |
|---|---|---|
| **Reconstitution Procedure** | | |
| **Sucrose Concentration (% w/v)** | **Time point (months)** | **Reconstitution Time (mins)** |
| **Humanized IgG1 at 100 mg/mL** | | |
| 0.1 | 0 | 33 |
| | 12 | 30 |
| 1 | 0 | 31 |
| | 12 | 32 |
| 2 | 0 | 29 |
| | 12 | 28 |
| 3 | 0 | 27 |
| | 12 | 30 |
| 5 | 0 | 28 |
| | 12 | 29 |
| 8.5 | 0 | 25 |
| | 12 | 27 |
| 10 | 0 | 26 |
| | 12 | 25 |

| **Humanized IgG1 at 50 mg/mL** | | |
|---|---|---|
| 0.1 | 0 | 5 |
| | 12 | 6 |
| 0.5 | 0 | 5 |
| | 12 | 4 |
| 1 | 0 | 4 |
| | 12 | 5 |
| 2.5 | 0 | 5 |
| | 12 | 4 |
| 5 | 0 | 4 |
| | 12 | 4 |

| **Tn3-HSA Fusion Protein at 50 mg/mL** | | |
|---|---|---|
| 1 | 0 | 6 |
| | 12 | 5 |
| 2.5 | 0 | 5 |
| | 12 | 4 |
| 5 | 0 | 4 |
| | 12 | 4 |

### Example 2

### Thermal Characterization of the Pharmaceutical Compositions

Freeze Dry Microscopy (FDM) was used to determine the collapse temperature **(T_{c})** of the pharmaceutical compositions described in **Example 1,** (Patapoff, T.W. et al. (2002) "The Importance of Freezing on Lyophilization Cycle Development," BioPharm. 2002:16-21 and 71; Nail, S.L. et al. (2002) "Fundamentals of Freeze-Drying," Pharm. Biotechnol. 14:281-360; Angell, C.A. (1995) "Formation of Glasses from Liquids and Biopolymers," Science 267:1924-1935; Wolanczyk, J.P. (1989) "Differential Scanning Calorimetry Analysis of Glass Transitions," Cryo-Letters 10:73-76; Gibbs, et al. (1958) "Nature of the Glass Transition and the Glassy State," J. Chem. Phys. 28(3):373-383). The glass transition temperature (Tg') relates to the observation that as a liquid cools its viscosity increases, such that the liquid will exhibit solid-like mechanical properties even though it has not undergone a phase transition to solid *(i.e.,* **Tg'** is always lower than the freezing/melting temperature, **Tₘ).** The glass transition is a temperature range through which the viscosity of a liquid changes upon cooling from its initial viscosity to the lowest viscosity of that liquid. The reported value **Tg'** is the temperature at which 50% of this change in viscosity has occurred. The collapse temperature **(T_{c})** is the lowest temperature at which a liquid containing multiple components can retain such components in soluble form and the highest temperature that a solid composed of multiple components can withstand without collapsing. At a temperature below the collapse temperature **(T_{c})** one or more of the components of the liquid will solidify out of the solution; at a temperature above the collapse temperature **(T_{c})** one or more of the components will liquefy or collapse.

**Figure 1** shows melting temperatures, **Tₘ₁** and **Tₘ₂** for the humanized IgG1 antibody. The curves show two **Tₘ's,** reflecting the melting of the Fc domain **(Tₘ₁)** and the melting of the Fab domain **(Tₘ₂).** The data show super-positioned curves representing the nearly identical **Tₘ₁** and **Tₘ₂** observed for pharmaceutical compositions with different sugar concentrations *(i.e., 0%,* 1%, 2%, 3%, 4% or 5% sucrose). The substantially identical nature of the curves indicates that the different sugar concentrations did not change the structures of the protein biomolecules.

**Figure 2** shows the observed collapse temperature **(Tc)** for the humanized IgG1 antibody. Similar trends were observed for **Tg'** for the humanized IgG1 antibody at concentrations of 50 mg/mL and at 100 mg/mL, and for **T_{g}'** for the Tn3-HSA fusion protein at a concentration of 50 mg/mL.

The **T_{c}** and **Tg'** values were found to increase with decreasing sucrose concentrations for compositions containing the humanized IgG1 antibody (at 50 mg/mL or 100 mg/mL). For the 100 mg/mL formulations, a significant increase in **T_{c}** (approximately 5 °C) was observed upon reducing the sugar concentration from 10% to 1% **(****Figure 2****),** which is the minimum sucrose concentration with stability of < 0.1% aggregation at 2-8 °C. A similar trend was observed for compositions containing the humanized IgG1 antibody (at 50 mg/mL). Here, reducing the sugar concentration from 5% to 1% resulted in an increase in **T_{c}** of approximately 7°C **(****Figure 2****).**

### Example 3

### Stability Study of Pharmaceutical Compositions Containing a Protein Biomolecule

Pharmaceutical compositions containing a humanized IgG1 antibody protein biomolecule at a concentration of 100 mg/mL were prepared as described in **Example 1.**

The pharmaceutical compositions were subjected to uncontrolled 1X freeze-thaw in a 100 mL PETG bottle containing approximately 90 mL of the pharmaceutical compositions **(****Figure 3****).** Freezing was performed at -80 °C and thawing was performed at room temperature.

High performance size-exclusion chromatography was used to measure aggregation. As shown in **Figure 3****,** the uncontrolled freeze-thaw did not affect monomer purity. Post-freeze-thaw, a visual inspection was performed in 3 cc glass vials with a 1 mL fill volume. The visual inspection of the vials for all protein-to-sugar ratio samples showed no change in visual appearance and no visible particle formation after 1X freeze-thaw stress. Pharmaceutical compositions comprising various levels of sucrose were additionally analyzed for sub-visible particles (SVP) in an HIAC liquid particle counter (HIAC). The analysis showed no increase in SVP as a consequence of the freeze-thaw.

Pharmaceutical compositions containing the humanized IgG1 antibody protein biomolecule at a concentration of 50 mg/mL or 100 mg/mL were prepared as described in **Example 1** and subjected to repeated controlled rate freeze-thawing cycles in vials and the impact of such treatment on aggregation was measured. The results of this investigation **(****Figures 4A-****4B)** show that preparations lacking sugar (and especially pharmaceutical compositions that contained 100 mg/mL concentrations of protein biomolecule, but lacked sugar) exhibited increased aggregation as a consequence of repeated freeze-thawing. The presence of sugar at concentrations of 1-4% (evaluated with respect to protein biomolecule concentrations of 50 mg/mL **(****Figure 4A****))** or at concentrations of 1-3% (evaluated with respect to protein biomolecule concentrations of 100 mg/mL **(****Figure 4B****))** was associated with monomer stability.

### Example 4

### Effect of Protein-to-Sugar Ratio on the Stability of Pharmaceutical Compositions Containing a Protein Biomolecule

Pharmaceutical compositions containing a humanized IgG1 antibody protein biomolecule at a concentration of 50 mg/mL or 100 mg/mL were prepared and lyophilized as described in **Example 1.** The lyophilisates were reconstituted at different time-points and the percent monomer purity of the reconstituted compositions was determined using high performance size-exclusion chromatography.

The pharmaceutical compositions were found to exhibit an aggregation rate that decreased with increased sugar concentration.

**Figure 5** shows the observed aggregation rate data for lyophilized product containing 100 mg/mL of the humanized IgG1 antibody protein biomolecule that had been stored at 60 °C for 7 days. **Figure 6** shows the observed aggregation rate data for reconstituted lyophilized compositions containing 50 mg/mL of the humanized IgG1 antibody protein biomolecule that had been stored at 40 °C (75% relative humidity) for 3 months or 25 °C (60% relative humidity) for 6 months prior to their reconstitution. **Figures 7A-7B** show the observed aggregation rate data for reconstituted lyophilized compositions containing 50 mg/mL **(****Figure 7A****)** or 100 mg/mL **(****Figure 7B****)** of the humanized IgG1 antibody protein biomolecule that had been stored at 2-8 °C for at least 12 months prior to reconstitution.

**Figures 8A-8B** compare the observed aggregation rates as a function of sugar concentration for liquid compositions containing 50 mg/mL and 100 mg/mL of the humanized IgG1 antibody protein biomolecule that had been stored for 2-8 °C for at least 12 months prior to reconstitution **(****Figure 8A****)** or at 40 °C (75% relative humidity) for 3 months **(****Figure 8B****).** The data show that the aggregation rate was higher for compositions that had been stored at 40 °C. However, the data show no significant difference in aggregation or liquid stability for compositions that had been stored at 2-8 °C and 40 °C in the presence of different sugar concentrations.

**Figure 9** shows the observed aggregation rates as a function of sugar concentration for reconstituted lyophilized compositions containing 50 mg/mL of the Tn3-HSA fusion protein biomolecule that had been stored for 40 °C (75% relative humidity) for 3 months or 25 °C (60% relative humidity) for 6 months prior to their reconstitution. **Figure 10** shows the observed aggregation rates as a function of sugar concentration for reconstituted lyophilized compositions containing 50 mg/mL of the Tn3-HSA fusion protein biomolecule that had been stored for 2-8 °C for at least 12 months prior to reconstitution.

### Example 5

### Effect of Protein to Sugar Ratio on the Long-Term Stability of Pharmaceutical Compositions Containing a Protein Biomolecule

In order to further evaluate the effect of sugar concentration on the solution stability of pharmaceutical compositions containing protein biomolecules, pharmaceutical compositions containing 50 mg/mL of the Tn3-HSA fusion protein, as an exemplary protein biomolecule, were stored at 2-8 °C for at least 12 months, at 40 °C (75% relative humidity) for 3 months, and aggregation rate was assessed. The results of this investigation are shown in **Figure 11****.** The data show that samples incubated at lower temperature exhibited substantially no aggregation, and that increased sugar concentration was associated with lower aggregation rates for material stored at higher temperature.

Table 6 shows the potency of the exemplary protein biomolecules upon storage at 2-8 °C for 12 or 24 months in the presence of differing concentrations of sucrose.

| **Table 6** | | |
|---|---|---|
| **Potency Data For Humanized IgG1 (at 50 or 100 mg/mL) or Tn3-HSA Fusion Protein (at 50 mg/mL) in Stored at 2-8 °C in Formulations Containing Different Levels of Sucrose** | | |
| **Sample (Lyophilized Drug Product)** | **Time Point (Months)** | **Bioassay Result (% Activity Relative to Reference Standard)** |
| **Humanized IgG1** | | |
| 100 mg/mL with 1% sucrose | 24 | 131 |
| 100 mg/mL with 2% sucrose | 24 | 123 |
| 100 mg/mL with 3% sucrose | 24 | 133 |
| 50 mg/mL with 0% sucrose | 12 | 128 |
| 50 mg/mL with 0.5% sucrose | 12 | 118 |
| 50 mg/mL with 1% sucrose | 12 | 106 |

| **Tn3-HSA-Fusion Protein** | | |
|---|---|---|
| 50 mg/mL with 1% sucrose | 12 | 99 |
| 50 mg/mL with 2.5% sucrose | 12 | 103 |
| 50 mg/mL with 5% sucrose | 12 | 102 |

The results indicate that the exemplary protein biomolecules exhibited exceptional stability upon storage in the formulations of the present invention.

### Example 6

### Preferred Pharmaceutical Compositions Containing a Protein Biomolecule

As shown above, pharmaceutical compositions containing high concentrations of a protein biomolecule showed aggregation as the major route of degradation on stability at 40 °C. Fragmentation of the protein biomolecule was also observed, but aggregation was the major route of degradation.

At 40 °C, both of the exemplary protein biomolecules evaluated showed exponential decreases in aggregation rates (at both 50 mg/mL and 100 mg/mL) as the sucrose concentration of the pharmaceutical composition was increased. Likewise at 25 °C, both exemplary protein biomolecules showed exponential decreases in aggregation rates with an increase in sucrose concentration.

In summary, pharmaceutical compositions comprising protein biomolecules, at both 50 mg/mL and at 100 mg/mL, with protein-to-sugar ratio of 1:0.1 *(i.e.,* 0.5% sugar for protein biomolecule concentrations of 50 mg/mL, and 1% sugar for protein biomolecule concentrations of 100 mg/mL) resulted in a shelf-life of 2-3 years at 2-8 °C storage. All formulations showed elegant cake structure without major defects regardless of the protein to sugar ratio. Moreover, all formulations upon reconstitution showed no visible particle formation. Sub-visible particle (SVP) analysis by HIAC showed no significant increase in SVP counts. Karl Fischer analysis for percent residual moisture in the lyophilisates showed <1% water content for all formulations. Also, regardless of the composition's sugar concentration, bioassay testing of lyophilized pharmaceutical compositions containing 50 mg/mL or 100 mg/mL of protein biomolecules that had been stored at 2-8 °C showed no significant differences in potency for up to 12 months of storage. Likewise, regardless of the composition's sugar concentration, bioassay testing of lyophilized pharmaceutical compositions containing 50 mg/mL of the Tn3-HSA fusion protein that had been stored at 2-8 °C showed no significant difference in potency with different sugar levels for up 12 months of storage. **Figures 12A-12C** show the visual appearance of the lyophilized cake for humanized IgG1 (at 50 and 100 mg/mL) and Tn3-HSA fusion protein (at 50 mg/mL) in formulations containing different levels of sucrose.

In conclusion, reducing the sucrose concentration of pharmaceutical compositions that contain high concentrations of protein biomolecules (for example, 100 mg/mL of the IgG1 humanized antibody) to 1% sucrose (w/v) *(i.e.,* a protein biomolecule to sugar ratio = 1:0.1 (w/w)) resulted in a significant increase (by approximately 5 °C) in T_{c} along with acceptable long-term stability at 2-8 °C.

Moreover, lowering the sucrose concentration of pharmaceutical compositions that contain lower, but still high, concentrations of protein biomolecules (for example, 50 mg/mL of the IgG1 humanized antibody) to 0.5% sucrose (w/v) *(i.e.,* a protein biomolecule to sugar ratio = 1:0.1 (w/w) resulted in a significant increase (by approximately 9 °C) in T_{c} without impacting long-term stability at 2-8 °C. Pharmaceutical compositions that contained 50 mg/mL of the Tn3-HSA fusion protein and a minimum 1% sucrose concentration *(i.e.,* a protein biomolecule to sugar ratio = 1:0.2 (w/w)) also showed acceptable long-term stability at 2-8 °C.

In summary, acceptable long-term stability at 2-8 °C of lyophilized pharmaceutical compositions containing high concentrations of protein biomolecules can be achieved even with a protein-to-sugar ratio of less than 1:1 (w/w). Reducing the sugar concentration of pharmaceutical compositions improves their thermal characteristics (T_{c} and Tg'), and can thereby reduce the lyophilization cycle times of the compositions. For designing the primary drying step, typically, T_{c} is used as the maximum allowable product temperature (Tₚₘₐₓ < T_{c}) (Colandene, J.D. et al (2007), "Lyophilization cycle development for a high-concentration monoclonal antibody formulation lacking a crystalline bulking agent", J Pharm Sci 96: 1598-1608. Typically, a 1 °C increase in the product temperature results in a 13% reduction in primary drying time (Tang, X et al. (2004) "Design of Freeze-Drying Processes for Pharmaceuticals: Practical Advice," Practical Advice. Pharm Res 21(2):191-200; Carpenter, J.F. et al. (2002) "Rational Design Of Stable Lyophilized Protein Formulations: Theory And Practice," Pharm. Biotechnol. 13:109-133) and primary drying is the longest step of the lyophilization cycle. As shown in Example 1 (Figure 2) and Table 7, reducing the sugar concentration in the protein formulation (100 mg/mL) resulted in ≥ 5°C increase in T_{c}. This increase in Tₚₘₐₓ results in around a 73% reduction in the primary drying step. Furthermore, reducing the sugar concentration in the protein formulation (50 mg/mL) resulted in ≥ 10°C increase in T_{c.} This increase in Tₚₘₐₓ results in around a 91% reduction in the primary drying step. Therefore, a significant reduction in lyophilization cycle time can be achieved by lowering the amount of sugar in the pharmaceutical composition without impacting drug product stability.

**Table 7: Primary drying time determination based on collapse temperature (T_{c}) for various protein-to-sugar ratios of an exemplary human IgG1 monoclonal antibody**

| **Protein conc. (mg/mL)** | **Sugar conc. (% w/v)** | **P:S ratio** | **Tₚₘₐₓ ~ T_{c}* (°C)** | **Approximate Primary Drying time (%)**** |
|---|---|---|---|---|
| **100** | **10** | **1:1** | **-15** | **100 (ref.)** |
| | **8.5** | **1:0.85** | **-13** | **74** |
| | **5** | **1:0.5** | **-11** | **48** |
| | **3** | **1:0.3** | **-11** | **48** |
| | **1** | **1:0.1** | **-9.5** | **28.5** |
| **50** | **5** | **1:1** | **-19.5** | **100 (ref.)** |
| | **2.5** | **1:0.5** | **-13.5** | **22** |
| | **1** | **1:0.2** | **-12.5** | **9** |
| | **0.5** | **1:0.1** | **-10** | **<9** |

Table 7 shows the approximate primary drying time (%) for formulations of an exemplary human IgG1 monoclonal antibody with different protein to sugar ratios. Primary drying time (%) is determined based on the rule that every 1°C increase in product temperature (Tₚ) results in a 13% reduction in primary drying time (Tang, X. et al. (2004) "Design of Freeze-Drying Processes for Pharmaceuticals: Practical Advice," Pharm Res. 21:191-200). The maximum allowable temperature (Tₚₘₐₓ) ~ collapse temperature (T_{c}) is calculated from Depaz, RA. et al. (2015) "Freeze-Drying Above the Glass Transition Temperature in Amorphous Protein Formulations While Maintaining Product Quality and Improving Process Efficiency," J Pharm Sci. 10.1002/jps.24705 and Colandene, JD. et al. (2007) "Lyophilization cycle development for a high-concentration monoclonal antibody formulation lacking a crystalline bulking agent," J Pharm Sci. 96:1598-1608. Therefore,

All publications and patents mentioned in this specification are herein incorporated by reference to the same extent as if each individual publication or patent application was specifically and individually indicated to be incorporated by reference in its entirety. While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth.

Also disclosed are the following aspects
1. A pharmaceutical composition comprising a protein biomolecule as an active agent or component thereof, wherein:
   (A) said composition is an aqueous solution that comprises:
      (1) a protein biomolecule;
      (2) a buffer; and
      (3) an amorphous stabilizing compound;
         wherein in said aqueous solution:
         (a) said protein biomolecule is present at a concentration of about 50 mg/mL or less, and said amorphous stabilizing compound is present at a total concentration of from about 0.1% (w/v) to about 2.5% (w/v); or
         (b) said protein biomolecule is present at a concentration of greater than about 50 mg/mL, and said amorphous stabilizing compound is present at a total concentration of from about 1% (w/v) to about 8.5% (w/v);
      or
   (B) said composition is a lyophilisate of said aqueous solution that comprises said protein biomolecule at a concentration of about 50 mg/mL or less.
2. The pharmaceutical composition of aspect 1, wherein said protein biomolecule is present in said aqueous solution at a concentration of about 50 mg/mL or less, and said amorphous stabilizing compound is present at a total concentration of from about 0.1% (w/v) to about 2.5% (w/v).
3. The pharmaceutical composition of aspect 2, wherein said amorphous stabilizing compound is present at a total concentration of about 0.5% (w/v).
4. The pharmaceutical composition of aspect 2, wherein said amorphous stabilizing compound is present at a total concentration of about 1% (w/v).
5. The pharmaceutical composition of any one of aspects 1-4, wherein said pharmaceutical composition is said lyophilisate.
6. The pharmaceutical composition of aspect 1, wherein said pharmaceutical composition is said aqueous solution, and wherein said protein biomolecule is present in said composition at a concentration of greater than about 50 mg/mL to about 500 mg/mL, and said amorphous stabilizing compound is present at a total concentration of from about 1% (w/v) to about 8.5% (w/v).
7. The pharmaceutical composition of aspect 6, wherein said protein biomolecule is present in said composition at a concentration of about 100 mg/mL, and said amorphous stabilizing compound is present at a total concentration of from about 1% (w/v) to about 8.5% (w/v).
8. The pharmaceutical composition of aspect 7, wherein said amorphous stabilizing compound is present at a total concentration of about 1% (w/v).
9. The pharmaceutical composition of aspect 6, wherein said protein biomolecule is present in said composition at a concentration of about 200 mg/mL, and said amorphous stabilizing compound is present at a total concentration of from about 1% (w/v) to about 8.5% (w/v).
10. The pharmaceutical composition of aspect 9, wherein said amorphous stabilizing compound is present at a total concentration of about 2% (w/v).
11. The pharmaceutical composition of any one of aspects 1-10, wherein said amorphous stabilizing compound is a sugar.
12. The pharmaceutical composition of aspect 11, wherein said sugar is sucrose, trehalose, glucose, lactose or sorbitol, or a mixture thereof.
13. The pharmaceutical composition of any one of aspects 1-10, wherein said amorphous stabilizing compound is an amino acid.
14. The pharmaceutical composition of aspect 13, wherein said amino acid is arginine, alanine, lysine, proline or glycine, or a derivative or salt thereof, or any mixture thereof.
15. The pharmaceutical composition of any one of aspects 1-14, wherein the composition comprises at least two amorphous stabilizing compounds.
16. The pharmaceutical composition of aspect 15, wherein one of the at least two amorphous stabilizing compounds is a sugar and the other is an amino acid.
17. The pharmaceutical composition of any one of aspects 1-16, wherein the composition comprises at least two protein biomolecules.
18. The pharmaceutical composition of any one of aspects 1-17, wherein said protein biomolecule is an antibody or an antibody-based immunotherapeutic, enzyme, or a hormone/factor.
19. The pharmaceutical composition of any one of aspects 1-18, wherein said protein biomolecule is an antibody or an antibody-based immunotherapeutic, and said antibody is selected from the antibodies of **Table 1.**
20. The pharmaceutical composition of any one of aspects 1-18, wherein said protein biomolecule is a hormone/factor, and said hormone/factor is selected from the hormone/factors of **Table 2.**
21. The pharmaceutical composition of any one of aspects 1-20, wherein the pH of said pharmaceutical composition is from about 3 to about 11, from about 4 to about 9, from about 5 to about 8, from about 5 to about 7.5, preferably 6.0 or 7.4.
22. The pharmaceutical composition of any one of aspects 1-21, wherein said buffer is present in a range of from about 1 mM to 100 mM, about 10 mM to about 50 mM, about 20 mM to about 30 mM, or about 23 mM to about 27 mM.
23. The pharmaceutical composition of any one of aspects 1-22, wherein said buffer comprises histidine, phosphate, acetate, citrate, succinate, Tris, or a combination thereof.
24. The pharmaceutical composition of aspect 23, wherein said buffer is histidine / histidine-HCl.
25. The pharmaceutical composition of any one of aspects 1-24, wherein said pharmaceutical composition additionally comprises a non-ionic detergent.
26. The pharmaceutical composition of aspect25, wherein said non-ionic detergent is polysorbate-80 (PS-80).
27. The pharmaceutical composition of aspect 26, wherein said polysorbate-80 (PS-80) is present at a concentration of 0.02% (w/v).
28. The pharmaceutical composition of any one of aspects 1-27, wherein the primary drying time is reduced by 25%, by 30%, by 35%, by 40%, by 45%, by 50%, by 55%, by 60%, by 65%, by 70%, by 75%, by 80%, by 85%, by 90%, or by 95%.
29. An ampoule, vial, syringe, cartridge or sachette that contains the pharmaceutical composition of any one of aspects 1-28.
30. A method of treating a disease or disorder by administering the pharmaceutical composition of any one of aspects 1-28.
31. The pharmaceutical composition of any one of aspects 1-28 for use in medicine.

## Claims

1. A pharmaceutical composition comprising a protein biomolecule as an active agent or component thereof, wherein:
(A) said composition is an aqueous solution or a lyophilisate thereof that comprises:
(1) a protein biomolecule;
(2) a buffer; and
(3) an amorphous stabilizing compound;
wherein in said aqueous solution:
(a) said protein biomolecule is present at a concentration of about 50 mg/mL or less, and said amorphous stabilizing compound is present at a total concentration of from about 0.1% (w/v) to about 2.5% (w/v); or
(b) said protein biomolecule is present at a concentration of greater than about 50 mg/mL, and said amorphous stabilizing compound is present at a total concentration of from about 1% (w/v) to about 8.5% (w/v); or
(B) said composition is a lyophilisate of said aqueous solution that comprises said protein biomolecule at a concentration of about 50 mg/mL or less.

2. The pharmaceutical composition according to claim 1, wherein said protein biomolecule is an antibody, wherein said composition is an aqueous solution or a lyophilisate thereof that comprises:
(1) said protein biomolecule
(2) a histidine buffer; and
(3) an amorphous stabilizing compound that is sucrose;
wherein in said aqueous solution:
(a) said protein biomolecule is present at a concentration of about 50 mg/mL, and said amorphous stabilizing compound is present at a total concentration of from about 0.1% (w/v) to about 2.5% (w/v);
or
(b) said protein biomolecule is present at a concentration of about 100 mg/mL, and said amorphous stabilizing compound is present at a total concentration of from about 1% (w/v) to about 8.5% (w/v).

3. The pharmaceutical composition of claim 1 or claim 2, wherein the composition comprises at least two amorphous stabilizing compounds.

4. The pharmaceutical composition of claim 3, wherein one of the at least two amorphous stabilizing compounds is a sugar and the other is an amino acid.

5. The pharmaceutical composition of any one of claims 1-4, wherein the composition comprises at least two protein biomolecules.

6. The pharmaceutical composition of any one of claims 1-5, wherein said antibody is selected from 3F8, 8H9, Abagovomab, Abciximab, Actoxumab, Adalimumab, Adecatumumab, Aducanumab, Afelimomab, Afutuzumab, Alacizumab, Ald518, Alemtuzumab, Alirocumab, Altumomab, Amatuximab, Anatumomab, Mafenatox, Anifrolumab, Anrukinzumab, Apolizumab, Arcitumomab, Aselizumab, Atinumab, Atlizumab, Atorolimumab, Bapineuzumab, Basiliximab, Bavituximab, Bectumomab, Belimumab, Benralizumab, Bertilimumab, Besilesomab, Bevacizumab, Bezlotoxumab, Biciromab, Bimagrumab, Bivatuzumab, Blinatumomab, Blosozumab, Brentuximab, Briakinumab, Brodalumab, Canakinumab, Cantuzumab, Cantuzumab, Mertansine, Caplacizumab, Capromab, Carlumab, Catumaxomab, Cc49, Certolizumab, Cetuximab, Ch.14.18, Citatuzumab, Cixutumumab, Clazakizumab, Clivatuzumab, Conatumumab, Concizumab, Cr6261, Crenezumab, Dacetuzumab, Daclizumab, Dalotuzumab, Daratumumab, Demcizumab, Denosumab, Detumomab, Dorlimomab, Aritox, Drozitumab, Duligotumab, Dupilumab, Dusigitumab, Ecromeximab, Eculizumab, Edobacomab, Edrecolomab, Efalizumab, Efungumab, Eldelumab, Elotuzumab, Elsilimomab, Enavatuzumab, Enlimomab, Enokizumab, Enoticumab, Ensituximab, Epitumomab, Cituxetan, Epratuzumab, Erlizumab, Ertumaxomab, Etaracizumab, Etrolizumab, Evolocumab, Exbivirumab, Fanolesomab, Faralimomab, Farletuzumab, Fasinumab, Fbta05, Felvizumab, Fezakinumab, Ficlatuzumab, Figitumumab, Flanvotumab, Fontolizumab, Foravirumab, Fresolimumab, Fulranumab, Futuxiumab, Galiximab, Ganitumab, Gantenerumab, Gavilimomab, Gemtuzumab Ozogamicin, Gevokizumab, Girentuximab, Glembatumumab Vedotin, Golimumab, Gomiliximab, Guselkumab, Ibritumomab Tiuxetan, Icrucumab, Igovomab, Imab362, Imgatuzumab, Inclacumab, Indatuximab Ravtansine, Infliximab, Inolimomab, Inotuzumab Ozogamicin, Intetumumab, Ipilimumab, Iratumumab, Itolizumab, Ixekizumab, Keliximab, Labetuzumab, Lambrolizumab, Lampalizumab, Lebrikizumab, Lemalesomab, Lerdelimumab, Lexatumumab, Libivirumab, Ligelizumab, Lintuzumab, Lirilumab, Lodelcizumab, Lorvotuzumab, Lucatumumab, Lumiliximab, Mapatumumab, Margetuximab, Matuzumab, Mavrilimumab, Mepolizumab, Metelimumab, Milatuzumab, Minretumomab, Mitumomab, Mogamulizumab, Morolimumab, Motavizumab, Moxetumomab Pasudotox, Muromonab-CD3, Nacolomab Tafenatox, Namilumab, Naptumomab Estafenatox, Narnatumab, Natalizumab, Nebacumab, Necitumumab, Nerelimomab, Nesvacumab, Nimotuzumab, Nivolumab, Nofetumomab Merpentan, Ocaratuzumab, Ocrelizumab, Odulimomab, Ofatumumab, Olaratumab, Olokizumab, Onartuzumab, Ontuxizumab, Oportuzumab Monatox, Oregovomab, Orticumab, Otlertuzumab, Oxelumab, Ozanezumab, Ozaralizumab, Pagibaximab, Palivizumab, Panitumumab, Pankomab, Panobacumab, Parsatuzumab, Pascolizumab, Pateclizumab, Patritumab, Pembrolizumab, Pemtumomab, Perakizumab, Pertuzumab, Pexelizumab, Pidilizumab, Pinatuzumab Vedotin, Pintumomab, Placulumab, Polatuzumab Vedotin, Ponezumab, Pritoxaximab, Pritumumab, Pro 140, Quilizumab, Racotumomab, Radretumab, Rafivirumab, Ramucirumab, Ranibizumab, Raxibacumab, Regavirumab, Reslizumab, Rilotumumab, Rituximab, Robatumumab, Roledumab, Romosozumab, Rontalizumab, Rovelizumab, Ruplizumab, Samalizumab, Sarilumab, Satumomab Pendetide, Secukinumab, Seribantumab, Setoxaximab, Sevirumab, Sgn-CD19a, Sgn-CD33a, Sibrotuzumab, Sifalimumab, Siltuximab, Simtuzumab, Siplizumab, Sirukumab, Solanezumab, Solitomab, Sonepcizumab, Sontuzumab, Stamulumab, Sulesomab, Suvizumab, Tabalumab, Tacatuzumab Tetraxetan, Tadocizumab, Tanezumab, Taplitumomab Paptox, Tefibazumab, Telimomab, Tenatumomab, Teneliximab, Teprotumumab, Ticilimumab, Tigatuzumab, Tildrakizumab, Tnx-650, Tocilizumab, Toralizumab, Tositumomab, Tovetumab, Tralokinumab, Trastuzumab, Trbs07, Tremelimumab, Tucotuzumab Celmoleukin, Tuvirumab, Ublituximab, Urelumab, Urtoxazumab, Ustekinumab, Vantictumab, Vapaliximab, Vatelizumab, Vedolizumab, Veltuzumab, Vepalimomab, Vesencumab, Volociximab, Vorsetuzumab, Votumumab, Zalutumumab, Zatuximab, Ziralimumab and Zolimomab Aritox.

7. The pharmaceutical composition of any one of claims 1-6, wherein the pH of said aqueous solution is from about 3 to about 11, from about 4 to about 9, from about 5 to about 8, from about 5 to about 7.5, preferably 6.0 or 7.4.

8. The pharmaceutical composition of any one of claims 1-7, wherein said buffer is present in a range of from about 1 mM to 100 mM, about 10 mM to about 50 mM, about 20 mM to about 30 mM, or about 23 mM to about 27 mM.

9. The pharmaceutical composition of any one of claims 1 or 3-8, wherein said buffer comprises histidine, phosphate, acetate, citrate, succinate, Tris, or a combination thereof.

10. The pharmaceutical composition of any one of claims 1-9, wherein said pharmaceutical composition additionally comprises a non-ionic detergent.

11. The pharmaceutical composition of claim 10, wherein said non-ionic detergent is polysorbate-80 (PS-80).

12. The pharmaceutical composition of claim 11, wherein said polysorbate-80 (PS-80) is present at a concentration of 0.02% (w/v).

13. An ampoule, vial, syringe, cartridge or sachette that contains the pharmaceutical composition of any one of claims 1-12.

14. The pharmaceutical composition of any one of claims 1-12 for use in medicine.
